# EUROPEAN PATENT APPLICATION

(11) **EP 4 563 165 A1**
(43) Date of publication of application: **04.06.2025**
(21) Application number: 24216227.9
(22) Date of filing: 28.11.2024
(51) Int. Cl.: A61K 47/68, A61P 35/00, C07K 16/30

(54) **PSMA-STEAP1 DUAL VARIABLE DOMAIN IMMUNOGLOBULIN (DVD-IG) MOLECULES AND DRUG CONJUGATES**

(30) Priority: 30.11.2023 US 202363604884 P; 06.02.2024 US 202463550451 P
(71) Applicant: AbbVie Inc., North Chicago, IL 60064 (US)
(72) Inventor: Han, Edward, Gurnee 60064 (US); Phillips, Andrew C., Libertyville 60064 (US); Reilly, Regina M., Libertyville 60064 (US); Touw, Debra, Worcester 60064 (US); Liu, Zhihong, Gurnee 60064 (US)
(74) Representative: J A Kemp LLP

(57) **Abstract**

The present disclosure provides dual variable domain immunoglobulin (DVD-Ig) molecules that bind to human STEAP1 and human PSMA and their drug conjugates, and methods of using the same.

## Description

### CROSS-REFERENCE TO RELATED APPLICATIONS

This application claims the benefit of U.S. Provisional Application No. 63/604,884, filed November 30, 2023, and U.S. Provisional Application No. 63/550,451, filed February 6, 2024, the contents of which are hereby incorporated by reference in their entirety.

### SEQUENCE LISTING

The instant application contains a Sequence Listing (Sequence Listing N431260EP.xml; Size: 27,785 bytes; and Date of Creation: November 25, 2024), which has been submitted electronically and is hereby incorporated by reference in its entirety..

### BACKGROUND OF THE INVENTION

Prostate cancer is the second most diagnosed cancer and the fifth leading cause of cancer death among men worldwide. Currently there are no curative therapies available for metastatic prostate cancer.

PSMA (prostate specific membrane antigen) and STEAP1 (six transmembrane epithelial antigen of the prostate-1) are well characterized antigens overexpressed in metastatic prostate cancer with low expression in normal tissue.

PSMA plays a role in the metabolism of folates that may contribute to prostate cancer progression. PSMA expression is increased in metastatic and hormone refractory tumors and correlates with tumor aggressiveness, stage, recurrence, and castration resistance.

STEAP1 is an ion channel or transporter that modulates the concentration of small molecules, ions and nutrients. STEAP1 functions in cellular communication and in cell adhesion processes. STEAP1 is overexpressed in advanced and metastatic tumors, where it is associated with aggressive cell proliferation and invasion.

Antibody drug conjugates (ADCs) are a rapidly growing class of cancer drugs that combine the cell surface targeting properties of antibodies conjugated to potent cytotoxic drugs using chemical linkers. An advantage of ADCs is their ability to deliver toxic payloads to a tumor, reducing toxicity associated with systemic exposure to normal tissues.

There is a need for novel therapeutics combining binding agents of prostate cancer antigens to enhance tumor coverage, mitigate resistance, and drive durable response.

### BRIEF SUMMARY OF THE INVENTION

In some aspects, the present disclosure provides a dual-variable domain immunoglobulin (DVD-Ig) drug conjugate comprising the following structure: 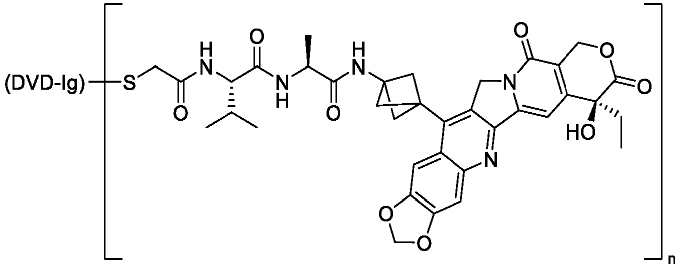
wherein n is 1 or 2,
wherein the DVD-Ig binds human STEAP1 and human PSMA,
wherein the DVD-Ig comprises two identical heavy chains and two identical light chains,
wherein each heavy chain comprises:
   a) a first heavy chain variable domain (VH1) comprising VH1-CDR1 (SEQ ID NO: 3), VH1-CDR2 (SEQ ID NO: 4), and VH1-CDR3 (SEQ ID NO: 5);
   b) a second heavy chain variable domain (VH2) a comprising VH2-CDR1 (SEQ ID NO: 8), VH2-CDR2 (SEQ ID NO: 9), and VH2-CDR3 (SEQ ID NO: 10); and
   c) an IgG1 heavy chain constant region (CH1CH2CH3);
wherein the VH1 and the VH2 are connected via a first peptide linker (L1), and each heavy chain is in the form of VH1-L1-VH2-CH1CH2CH3;
wherein each light chain comprises:
   a) a first light chain variable domain (VL1) comprising VL1-CDR1 (SEQ ID NO: 13), VL1-CDR2 (SEQ ID NO: 14), and VL1-CDR3 (SEQ ID NO: 15);
   b) a second light chain variable domain (VL2) comprising VL2-CDR1 (SEQ ID NO: 18), VL2-CDR2 (SEQ ID NO: 19), and VL2-CDR3 (SEQ ID NO: 20), and
   c) a kappa light chain constant region (Cκ);
wherein the VL1 and the VL2 are connected via a second peptide linker (L2), and each light chain is in the form of VL1-L2-VL2-Cx; and
wherein the first and the second heavy chains form a dimeric Fc region.

In some embodiments, the first heavy chain variable domain (VH1) comprises the amino acid sequence of SEQ ID NO: 2; the second heavy chain variable domain (VH2) comprises the amino acid sequence of SEQ ID NO: 7; the first light chain variable domain (VL1) comprises the amino acid sequence of SEQ ID NO: 12; and the second light chain variable domain (VL2) comprises the amino acid sequence of SEQ ID NO: 17.

In some embodiments, the first peptide linker (L1) comprises the amino acid sequence GGGGSGGGGS (SEQ ID NO: 6). In some embodiments, the second peptide linker (L2) comprises the amino acid sequence GGSGGGGSG (SEQ ID NO: 16). In some embodiments, the IgG1 heavy chain constant region (CH1CH2CH3) comprises the amino acid sequence of SEQ ID NO: 21 or 23. In some embodiments, the kappa light chain constant region (Cκ) comprises the amino acid sequence of SEQ ID NO: 22.

In some embodiments, each of the heavy chains comprise the amino acid sequence of SEQ ID NO: 1 or 24; and each light chain comprises the amino acid sequence of SEQ ID NO: 11.

In some embodiments, the first heavy chain and the second heavy chain are connected by two disulfide bridges, and the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering.

In some embodiments, the Top1i linker-Drugs are conjugated to the cysteines at position C220 of the first and the second heavy chains of the DVD-Ig according to EU numbering.

In some embodiments, n is 1. In some embodiments, n is 2.

In some aspects, the present disclosure provides a dual-variable domain immunoglobulin (DVD-Ig) drug conjugate comprising the following structure: 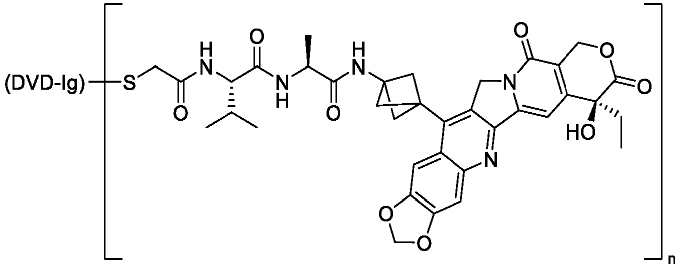
wherein n is 2, and wherein the DVD-Ig binds human STEAP1 and human PSMA, and comprises two identical heavy chains and two identical light chains,
wherein each of the heavy chains comprise the amino acid sequence of SEQ ID NO: 1 or 24; and
wherein each light chain comprises the amino acid sequence of SEQ ID NO: 11;
wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering; and
wherein the Top1i linker-Drugs are conjugated to the cysteines at position C220 of the first and the second heavy chains of the DVD-Ig according to EU numbering.

### BRIEF DESCRIPTION OF THE FIGURES

FIG. 1 is a diagram depicting the design of a DVD-Ig molecule, according to an embodiment of the invention.
FIG. 2A is a representative plot that shows STEAP1 and PSMA mRNA levels in a patient's tumor sample.
FIG. 2B is a diagram depicting the process and results of the screen to identify DVD-Ig molecules of interest.
FIGS. 3A - 3C show *in vitro* cytotoxicity data of eight PSMA-STEAP1 DVD-Igs (Clone 2, Clone 4, Clone 9, Clone 11, Clone 12, Clone 13, Clone 14, and Clone 15) conjugated with MMAE in LNCaP.FGC cells (FIG. 3A), HEK293-PSMA cells (FIG. 3B), and HEK293- STEAP1 cells (FIG. 3C).
FIGS. 3D - 3F show *in vitro* cytotoxicity data of PSMA-STEAP1 DVD-Ig clone 15 conjugated with MMAE ("DVD-MMAE") in LNCaP.FGC cells (FIG. 3D), HEK293-PSMA cells (FIG. 3E), and HEK293- STEAP1 cells (FIG. 3F).
FIGs. 4A and 4B show that DVD-MMAE demonstrated comparable or superior activity to either PSMA-MMAE or STEAP1-MMAE in 22Rv1 (FIG. 4A) and LNCaP.FGC (FIG. 4B) xenograft models. Surface expression of PSMA and STEAP1 is high in both models. DVD-MMAE: PSMA-STEAP1 DVD-Ig clone 15 conjugated with monomethyl auristatin E (MMAE); PSMA-MMAE: PSMA-targeting monoclonal, monospecific antibody conjugated with the MMAE; STEAP1-MMAE: STEAP1-targeting monoclonal, monospecific antibody conjugated with the MMAE.
FIG. 5A shows hydrophobic interaction chromatography (HIC) data.
FIG. 5B shows size exclusion chromatography (SEC) data.
FIGS. 6A-6C show ADC internalization and *in vitro* cytotoxicity for the LNCaP.FGC xenograft model. PSMA-STEAP1 DVD-Ig1 conjugated with Top1i at DAR 2 ("PSMA-STEAP1-Top1i") internalized faster (FIGS. 6A and 6B) and reduced cell viability more than either monospecific ADC (FIG. 6C). Payload accumulation was roughly 2x higher for PSMA-STEAP1-Top1i (FIG. 6B).
FIGs. 7A and 7B show that PSMA-STEAP1-Top1i has comparable or superior activity than that of individual ADCs (PSMA-Top1i and STEAP1-Top1i) in 22 Rv1 (FIG. 7A) and LNCaP.FGC (FIG. 7B) xenograft models.

### DETAILED DESCRIPTION OF THE INVENTION

In some aspects, described herein are novel DVD-Ig molecules that bind to both human STEAP1 and human PSMA, as well as drug conjugates of the said DVD-Ig molecules.

PSMA and STEAP1 are well characterized antigens overexpressed in metastatic prostate cancer with low expression in normal tissue. Both PSMA and STEAP1 are expressed over a range of intensities within prostate cancer cells. Such intratumor heterogeneity can hinder successful treatment of prostate cancer. In the present disclosure, the DVD-Ig platform is utilized for dual targeting of PSMA and STEAP1, which increases both breath and depth of responses relative to monospecific ADCs. Compared to monospecific ADCs, which target a single antigen PSMA or STEAP1, targeting both antigens offers several advantages: (1) dual targeting has broader efficacy on tumor cells and captures larger patient populations; (2) DVD-Ig molecules conjugated with cytotoxic drugs (DVD-Ig DCs) show enhanced internalization and activity than either monospecific ADC; and (3) compared to administration of a combination of two monospecific ADCs, DVD-Ig DCs reduce total dosing of agent(s) required.

DVD-Ig are symmetrical molecules with two identical light chains and two identical heavy chains and therefore have a more simplified production process than four-chain asymmetric formats that can suffer from chain mis-pairing and require downstream purification. However, identification of a DVD-Ig that is worthy of being a drug candidate, with the desired potency, physicochemical properties, efficacy, and manufacturability is challenging. The first challenge inherent to its design, is that the inner variable region can have reduced or eliminated binding affinity to its target. This is due to steric hindrance and can sometimes be mitigated by the choice of linker between the variable regions. A second challenge is that the expression level of some DVD-Igs can be lower than that of the parental IgG from which they are derived. Thirdly, the drug-like properties such as thermal stability, aggregation, or ability to achieve the high concentrations required for drug product formulation can be poor for some molecules. DVD-Igs described in the present disclosure have good expression level and strong binding specificity directed towards both human STEAP and human PSMA, and potent induction of cancer cell killing.

To obtain the DVD-Igs, 200 molecules were designed and cloned for expression. 149 molecules were successfully expressed, and 122 molecules had sufficient yield for characterization. 45 molecules had dual binding activity, and 20 were selected for additional characterization. A lead molecule was identified for further development, as it demonstrated good cell killing potency and acceptable drug-like properties.

### Definitions

The singular forms "a," "an" and "the" include plural referents unless the context clearly dictates otherwise.

The term "and/or" as used in a phrase such as "A and/or B" herein is intended to mean "A and B", "A or B", "A" or "B".

"Binding to human STEAP1 or human PSMA" refers to a molecule that is capable of binding human STEAP1 or human PSMA with sufficient affinity such that the molecule is useful as a therapeutic agent in targeting human STEAP or human PSMA.

The term "CDR" refers to a complementarity determining region within an immunoglobulin variable region sequence. There are three CDRs in each of the variable regions of the heavy chain and the light chain, which are designated CDR1, CDR2 and CDR3. The term "CDR set" refers to a group of three CDRs that are present in a single variable region capable of binding the antigen. The exact boundaries of these CDRs have been defined differently according to different systems. The system described by Kabat (Kabat et al. (1987) Sequences of Proteins of Immunological Interest, Fourth Edition. US Govt. Printing Off. No. 165-492; and Kabat et al. (1991) Sequences of Proteins of Immunological Interest, Fifth Edition. NIH Publication No. 91-3242) not only provides an unambiguous residue numbering system applicable to any variable region of an antibody or binding protein, but also provides precise residue boundaries defining the three CDRs in each heavy or light chain sequence. These CDRs may be referred to as Kabat CDRs. Chothia and coworkers (Chothia and Lesk (1987) J. Mol. Biol. 196:901-917; Chothia et al. (1989) Nature 342:877-883) found that certain sub-portions within Kabat CDRs adopt nearly identical peptide backbone conformations, despite having great diversity at the level of amino acid sequence. These sub-potions were designated as L1, L2 and L3 or H1, H2 and H3 where the "L" and the "H" designates the light chain and the heavy chain regions, respectively. These regions may be referred to as Chothia CDRs, which have boundaries that overlap with Kabat CDRs. Other boundaries defining CDRs overlapping with the Kabat CDRs have been described in Padlan (1995) FASEB J. 9:133-139 and MacCallum (1996) J. Mol. Biol. 262(5):732-45). Still other CDR boundary definitions may not strictly follow one of the herein described systems, but will nonetheless overlap with the Kabat CDRs, although they may be shortened or lengthened in light of prediction or experimental findings that particular residues or groups of residues or even entire CDRs do not significantly impact antigen binding. In some embodiments, the CDRs of the DVD-Igs of the present disclosure are defined by EU numbering.

The terms "Kabat numbering", "Kabat definition" and "Kabat labeling" are used interchangeably herein. These terms, which are recognized in the art, refer to a system of numbering amino acid residues which are more variable (i.e., hypervariable) than other amino acid residues in the heavy and light chain variable regions of an antibody or binding protein, or an antigen binding portion thereof (Kabat et al. (1971) Ann. NY Acad. Sci. 190:382-391 and, Kabat et al. (1991) Sequences of Proteins of Immunological Interest, Fifth Edition, U.S. Department of Health and Human Services, NIH Publication No. 91-3242). For the heavy chain variable region, the hypervariable region ranges from amino acid positions 31 to 35 for CDR1, amino acid positions 50 to 65 for CDR2, and amino acid positions 95 to 102 for CDR3. For the light chain variable region, the hypervariable region ranges from amino acid positions 24 to 34 for CDR1, amino acid positions 50-56 for CDR2, and amino acid positions 89 to 97 for CDR3.

In some embodiments, the constant region (heavy chain constant region and/or light chain constant region) sequences are identified using EU numbering. The term "EU numbering", which is recognized in the art, refers to a system of numbering amino acid residues based on Edelman, G.M. et al., Proc. Natl. Acad. USA, 63, 78-85 (1969). The numbering schemes disclosed herein, including EU numbering and Kabat numbering, are based on the conventional antibody structure. Because DVD-Ig has an extra variable domain (i.e., outer variable domain) connected to the N-terminal of the inner variable domain via a flexible linker, one of ordinary skill in the art would understand that the exact position of the amino acid based on the DVD-Ig structure would be different from the EU numbering or Kabat numbering. However, one of ordinary skill in the art would readily identify the corresponding amino acid within the DVD-Ig sequence based on the number schemes (e.g., EU numbering and Kabat numbering) disclosed herein. One of skill in the art could also identify positions of the DVD-Ig amino acid sequence by counting from the processed N-terminus, which does not conform to any numbering scheme.

The term "Fc" or "Fc region" is a term well known to the skilled artisan and is involved in complement activation, Clq binding, C3 activation and Fc receptor binding.

The term "hinge region" refers to a flexible amino acid stretch in the central part of the heavy chains of immunoglobulin antibodies, which links these 2 chains by disulfide bonds. The "variable domain" or "variable region" (variable domain/region of a light chain (VL), variable domain/region of a heavy chain (VH)) as used herein denotes each of the pair of light and heavy chains which are involved directly in binding the antibody to the target. The domains of variable human light and heavy chains have the same general structure and each domain comprises at least one complementary determining region (CDR), preferably three CDRs, which play a particularly important role in the binding specificity / affinity of the antibodies.

### DVD-Ig Molecules

The dual variable domain immunoglobulin (DVD-Ig) combines the target binding domains of two monoclonal antibodies (mAbs) via a flexible linker, which yields a tetravalent IgG-like molecule. DVD-Ig is symmetrical, bispecific antibody-like biologics with a 2 + 2 antigen binding stoichiometry. In some embodiments, in a linear representation of the mature sequence, the DID-Ig comprises an outer variable domain (VH1 or VL1) connected to the inner variable domain (VH2 or VL2) via a linker (L), with a standard Heavy Chain (HC) or Light Chain (LC) constant region at the C-terminus.

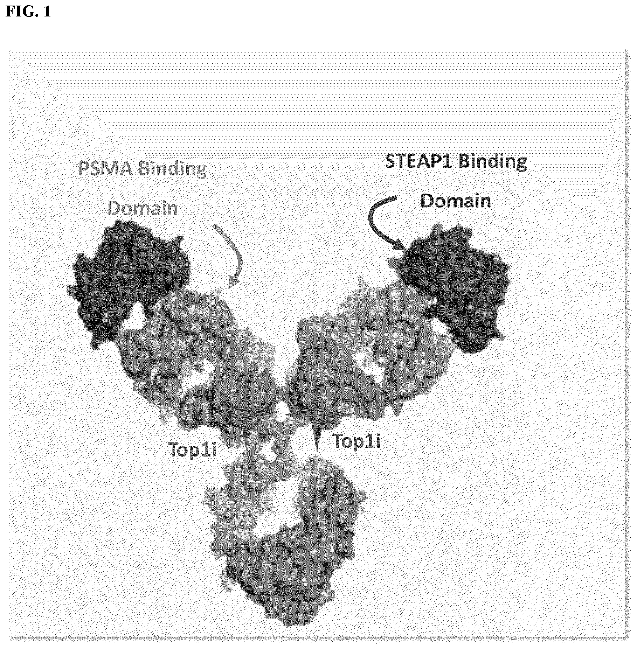

In some embodiments, the DVD-Ig provided herein comprises outer variable domains (VH1 and VL1) that bind human STEAP1 and inner variable domains (VH2 and VL2) that bind human PSMA. The diagram depicting the design of DVD-Ig molecule, according to an embodiment of the invention, is shown in FIG. 1.

For example, in some aspects, the present disclosure provides a DVD-Ig that binds human STEAP1 and human PSMA comprising two identical heavy chains and two identical light chains,
wherein each heavy chain comprises:
   a) a first heavy chain variable domain (VH1) comprising VH1-CDR1 (SEQ ID NO: 3), VH1-CDR2 (SEQ ID NO: 4), and VH1-CDR3 (SEQ ID NO: 5);
   b) a second heavy chain variable domain (VH2) a comprising VH2-CDR1 (SEQ ID NO: 8), VH2-CDR2 (SEQ ID NO: 9), and VH2-CDR3 (SEQ ID NO: 10); and
   c) an IgG1 heavy chain constant region (CH1CH2CH3);
wherein the VH1 and the VH2 are connected via a first peptide linker (L1), and each heavy chain is in the form of VH1-L1-VH2-CH1CH2CH3;
wherein each light chain comprises:
   a) a first light chain variable domain (VL1) comprising VL1-CDR1 (SEQ ID NO: 13), VL1-CDR2 (SEQ ID NO: 14), and VL1-CDR3 (SEQ ID NO: 15);
   b) a second light chain variable domain (VL2) comprising VL2-CDR1 (SEQ ID NO: 18), VL2-CDR2 (SEQ ID NO: 19), and VL2-CDR3 (SEQ ID NO: 20), and
   c) a kappa light chain constant region (Cκ);
wherein the VL1 and the VL2 are connected via a second peptide linker (L2), and each light chain is in the form of VL1-L2-VL2-Cx; and
wherein the first and the second heavy chains form a dimeric Fc region.

In some embodiments, the first heavy chain variable domain (VH1) comprises the amino acid sequence of SEQ ID NO: 2; the second heavy chain variable domain (VH2) comprises the amino acid sequence of SEQ ID NO: 7; the first light chain variable domain (VL1) comprises the amino acid sequence of SEQ ID NO: 12; and the second light chain variable domain (VL2) comprises the amino acid sequence of SEQ ID NO: 17.

In some embodiments, each of the heavy chains comprise the amino acid sequence of SEQ ID NO: 1 or 24; and each light chain comprises the amino acid sequence of SEQ ID NO: 11.

Flexible Glycine-Serine rich linkers are preferred for DVD-Igs. The GS linkers can be for DVD-Igs of the present disclosure include, but are not limited to, the ones listed in the table below. In some embodiments, the linker (e.g., the linker connecting VH1 and VH2, or L1) comprises GGGGSGGGGS (SEQ ID NO: 6). In some embodiments, the linker (e.g., the linker connecting VL1 and VL2, or L2) comprises GGSGGGGSG (SEQ ID NO: 16).

**Table 2**

| Linker Length (AA) | Linker Name | Sequence | SEQ ID NO |
|---|---|---|---|
| 6 | HC-GS6 | GGGGSG | 25 |
| 10 | HC-GS9 | GGGGSGGGGS | 6 |
| 14 | HC-GS 14 | GGGGSGGGGSGGGG | 26 |
| 5 | LC-GS5 | GGSGG | 27 |
| 9 | LC-GS9 | GGSGGGGSG | 16 |
| 13 | LC-GS 13 | GGSGGGGSGGGGS | 28 |

In some embodiments, the kappa light chain constant region (Cκ) is a human Cκ or is derived from a human Cκ region. In some embodiments, the LC cysteine that normally forms the interchain disulfide bond with C220 of the heavy chain (HC) has been mutated to alanine, which creates an unpaired cysteine at position C220 of the HC for conjugation of the linker drug in a facile, site-specific manner. For example, in some embodiments, the kappa light chain constant region (Cκ) is derived from the human Cκ and comprises an amino acid substitution of C214A according to EU numbering of the amino acid number of the antibody chain.

In some embodiments, the kappa light chain constant region (Cκ) comprises the amino acid sequence of SEQ ID NO: 22.

In one embodiment, CH2CH3 of the first and the second heavy chains form a dimeric Fc region. In one embodiment, the Fc region of the present invention is derived from human origin. In one embodiment, the Fc region of the present invention is a human IgG1 Fc region or derived from a human IgG1 Fc region.

In some embodiments, the Fc region comprises a first CH2CH3 region and a second CH2CH3 region, wherein the first CH2CH3 region is covalently bound by two disulfide bridges to the second CH2CH3 region. In some embodiments, the first heavy chain and the second heavy chain are connected by two disulfide bridges, and the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering. For example, in some embodiments, the first heavy chain and the second heavy chain have an amino acid sequence of SEQ ID NO: 1 or 24, and the two disulfide bridges are between C362 of the first heavy chain of SEQ ID NO: 1 or 24 and C362 of the second heavy chain of SEQ ID NO: 1 or 24, and between C365 of the first heavy chain of SEQ ID NO: 1 or 24 and C365 of the second heavy chain of SEQ ID NO: 1 or 24.

In one embodiment, the Fc region of the present invention comprises a hinge region. Various hinge regions can be used in the bispecific molecules of the present invention, for example, to optimize certain characteristics. In an illustrative example, one or more amino acid substitutions, insertions, and/or deletions within a hinge region of a human IgG₁, IgG₂, IgG₃ or IgG₄ can be introduced to reduce the level or rate of fragmentation and/or aggregation.

In one embodiment, the Fc region of the present invention comprises one or more mutations to modulate (e.g., reduce) IgG Fc (Fragment crystallizable) receptor and complement binding. In one embodiment, the Fc region of the present invention comprises one or more mutations to modulate (e.g., reduce) Fc receptor-based function of the Fc region. In one embodiment, the Fc region of the present invention comprises one or more mutations to modulate (e.g., reduce) FcyR-based effector function of the Fc region. In one embodiment, the Fc region of the present invention is derived from the human IgG1 Fc region and comprises an amino acid substitution of L234A and/or L235A according to EU numbering of the amino acid number of the antibody chain.

In one embodiment, the CH1CH2CH3 of the present invention comprises the amino acid sequence of SEQ ID NO: 21 or 23.

In some embodiments, the polypeptide sequence of either or both heavy chains of the DVD-Ig molecules described herein lack the C-terminal lysine, resulting in a C-terminal glycine residue. For example, in some embodiments, either or both heavy chains of the DVD-Ig molecules described herein comprise the amino acid sequence of SEQ ID NO: 24.

In some aspects, the present disclosure provides a DVD-Ig that binds human STEAP1 and human PSMA comprising two identical heavy chains and two identical light chains, wherein each of the heavy chains comprise the amino acid sequence of SEQ ID NO: 1 or 24, and each light chain comprises the amino acid sequence of SEQ ID NO: 11; and wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering.

### DVD-Ig Drug Conjugates (DVD-Ig DCs)

In some embodiments, DVD-Ig molecules described herein are conjugated to a drug, e.g., a Topoisomerase 1 Inhibitor (TOP1i). Topoisomerase 1 (TOP1) removes supercoils formed during DNA replication. TOP1 inhibitors (TOP1i) can bind and stabilize TOP1-DNA complexes, inducing DNA strand breakage and apoptosis. In some embodiments, DVD-Ig molecules described herein are conjugated to a Topoisomerase 1 inhibitor drug ("TOP1i drug) according to the structural formula (I).

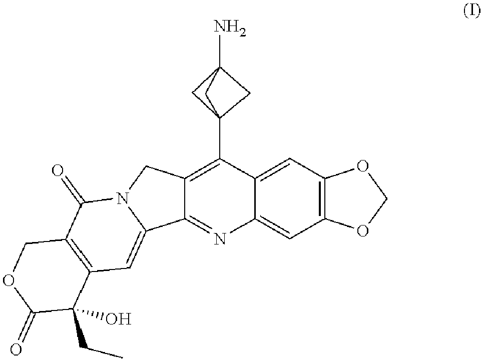

In some embodiments, a TOP1i drug is a compound according to formula (I). In embodiments, the TOP1i drug is (7S)-14-(3-aminobicyclo [1.1.1]pentan-1-yl)-7-ethyl-7-hydroxy-2H, 10H-[1,3]dioxolo[4,5-g]pyrano[3',4': 6,7]indolizino[1,2-b]quinoline-8,11 (7H,13H)-dione.

TOP1i drugs as contemplated herein may be conjugated to a DVD-Ig described herein as DVD-Ig DC as shown in structural formula (II): 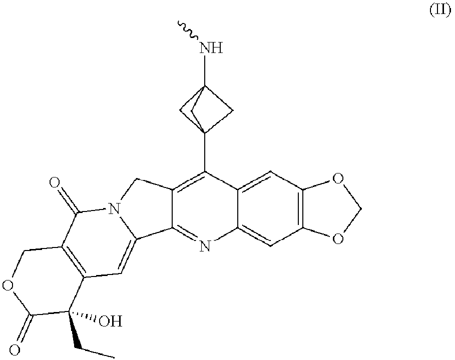 wherein 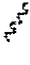 represents the point of attachment of a linker to the TOP1i drug.

Topoisomerase inhibitors as described herein may be conjugated to DVD-Ig described herein to form a DVD-Ig TOP1i drug conjugate. DVD-Ig-drug conjugates may increase the therapeutic efficacy of DVD-Ig in treating disease due to the ability of the DVD-Ig DC to selectively deliver one or more drug moiety(s) to target tissues, such as a tumor-associated antigen, e.g., human STEAP1 and/or human PSMA expressing tumors. Thus, in embodiments, the present disclosure provides DVD-Ig DCs described herein for therapeutic use, e.g., in the treatment of prostate cancer.

In certain embodiments, a TOP1i drug is conjugated to an antibody by way of linker according to structural formula (III): 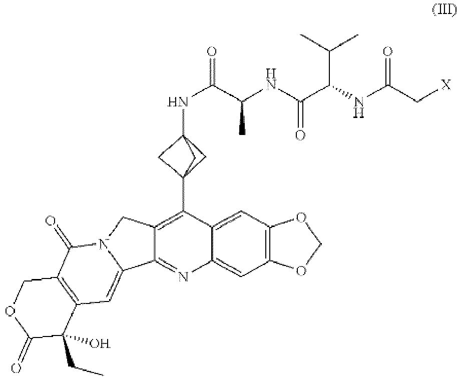 wherein X represents bromo or an N-linked maleimide.

In some embodiments of the DVD-Ig DCs described herein, TOP1i drugs are conjugated to the DVD-Igs by way of a linker moiety. As will be appreciated by skilled artisans, the linkers connect the TOP1i drug to the DVD-Ig by forming a covalent linkage to the TOP1i drug at one location and a covalent linkage to the antibody at another. The covalent linkages are formed by reaction between functional groups on the linker and functional groups on the TOP1i drug and the DVD-Ig. In some embodiments, the Top1i linker-Drugs are conjugated to the cysteines at position C220 of the first and the second heavy chains of the DVD-Ig described herein.

In some embodiments, the synthetic intermediate compounds that may be used to form DVD-Igs may include the Linker Drug (LD) shown as in Structural Formula (V))

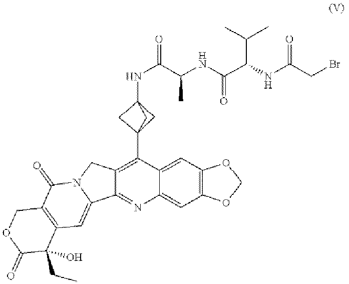

(2S)-2-(2-bromoacetamido)-N-[(2S)-1-({3-[(7S)-7-ethyl-7-hydroxy-8,11-dioxo-7,8,11,13-tetrahydro-2H,10H-[1,3]dioxolo[4,5-g]pyrano[3',4':6,7]indolizino[1,2-b]quinolin-14-yl]bicyclo[1.1.1]pentan-1-yl}amino)-1-oxopropan-2-yl]-3-methylbutanamide

The DVD-Ig DCs disclosed herein comprise drug molecules linked to DVD-Ig moieties in various stoichiometric molar ratios depending on the configuration of the DVD-Ig and, at least in part, the method used to effect conjugation.

The terms "drug load" or "drug loading" refer to the number of drug molecules per antibody in an individual DVD-Ig DC molecule. The number of TOP1i drugs linked to an DVD-Ig DC can vary and will be limited by the number of available attachments sites on the DVD-Ig. As contemplated for the DVD-Ig DCs of the invention, the linker will link a single TOP1i drug to a DVD-Ig. In some embodiments, the DVD-Ig DCs described herein have an n of 1 or 2. In embodiments, n is 1. In embodiments, n is 2. In embodiments, the drug loading may comprise 1 drug molecule, or 2 drug molecules.

In some aspects, the present disclosure provides a dual-variable domain immunoglobulin (DVD-Ig) drug conjugate comprising the following structure: 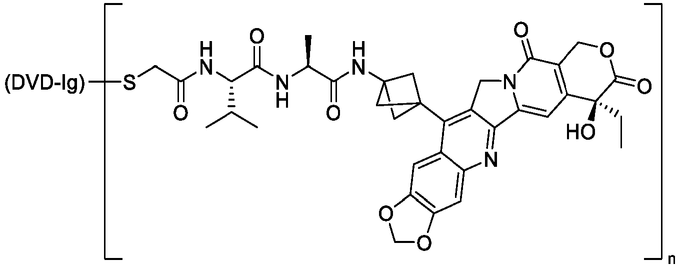
wherein n is 1 or 2, and wherein DVD-Ig binds human STEAP1 and human PSMA, and comprises two identical heavy chains and two identical light chains,
wherein each heavy chain comprises:
   a) a first heavy chain variable domain (VH1) comprising VH1-CDR1 (SEQ ID NO: 3), VH1-CDR2 (SEQ ID NO: 4), and VH1-CDR3 (SEQ ID NO: 5);
   b) a second heavy chain variable domain (VH2) a comprising VH2-CDR1 (SEQ ID NO: 8), VH2-CDR2 (SEQ ID NO: 9), and VH2-CDR3 (SEQ ID NO: 10); and
   c) an IgG1 heavy chain constant region (CH1CH2CH3);
wherein the VH1 and the VH2 are connected via a first peptide linker (L1), and each heavy chain is in the form of VH1-L1-VH2-CH1CH2CH3;
wherein each light chain comprises:
   a) a first light chain variable domain (VL1) comprising VL1-CDR1 (SEQ ID NO: 13), VL1-CDR2 (SEQ ID NO: 14), and VL1-CDR3 (SEQ ID NO: 15);
   b) a second light chain variable domain (VL2) comprising VL2-CDR1 (SEQ ID NO: 18), VL2-CDR2 (SEQ ID NO: 19), and VL2-CDR3 (SEQ ID NO: 20), and
   c) a kappa light chain constant region (Cκ);
wherein the VL1 and the VL2 are connected via a second peptide linker (L2), and each light chain is in the form of VL1-L2-VL2-Cx; and
wherein the first and the second heavy chains form a dimeric Fc region.

In some embodiments, the first heavy chain variable domain (VH1) comprises the amino acid sequence of SEQ ID NO: 2; the second heavy chain variable domain (VH2) comprises the amino acid sequence of SEQ ID NO: 7; the first light chain variable domain (VL1) comprises the amino acid sequence of SEQ ID NO: 12; and the second light chain variable domain (VL2) comprises the amino acid sequence of SEQ ID NO: 17.

In some embodiments, each heavy chain comprises the amino acid sequence of SEQ ID NO: 1 or 24; and each light chain comprises the amino acid sequence of SEQ ID NO: 11.

In some embodiments, the first peptide linker (L1) comprises the amino acid sequence GGGGSGGGGS (SEQ ID NO: 6). In some embodiments, the second peptide linker (L2) comprises the amino acid sequence GGSGGGGSG (SEQ ID NO: 16).

In some embodiments, the IgG1 heavy chain constant region (CH1CH2CH3) comprises the amino acid sequence of SEQ ID NO: 21 or 23. In some embodiments, the kappa light chain constant region (Cκ) comprises the amino acid sequence of SEQ ID NO: 22.

In some embodiments, the first heavy chain and the second heavy chain are connected by two disulfide bridges, and the two disulfide bridges are between the cysteine in position 226 of the first heavy chain and the cysteine in position 226 of the second heavy chain, and between the cysteine in position 229 of the first heavy chain and the cysteine in position 229 of the second heavy chain, wherein the amino acid positions are numbered using EU numbering. For example, in some embodiments, the first heavy chain and the second heavy chain have an amino acid sequence of SEQ ID NO: 1 or 24, and the two disulfide bridges are between C362 of the first heavy chain of SEQ ID NO: 1 or 24 and C362 of the second heavy chain of SEQ ID NO: 1 or 24, and between C365 of the first heavy chain of SEQ ID NO: 1 or 24 and C365 of the second heavy chain of SEQ ID NO: 1 or 24.

In some embodiments, the Top1i linker-Drugs are conjugated to the cysteines at position C220 of the first and the second heavy chains of the DVD-Ig according to EU numbering.

In some embodiments, n is 1.

In some embodiments, n is 2.

In some aspects, the present disclosure provides a dual-variable domain immunoglobulin (DVD-Ig) drug conjugate comprising the following structure: 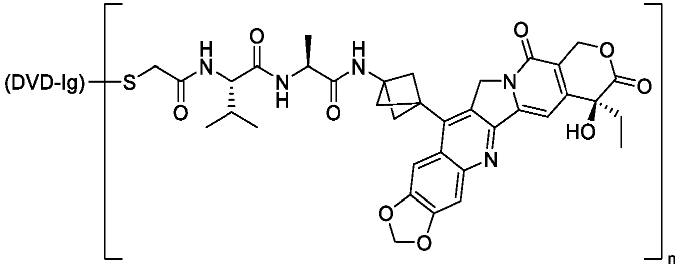
wherein n is 2,
wherein DVD-Ig binds human STEAP1 and human PSMA, and comprises two identical heavy chains and two identical light chains,
wherein each of the heavy chains comprise the amino acid sequence of SEQ ID NO: 1 or 24; and
wherein each light chain comprises the amino acid sequence of SEQ ID NO: 11;
wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering; and
wherein the Top1i linker-Drugs are conjugated to the cysteines at position C220 of the first and the second heavy chains of the DVD-Ig according to EU numbering.

### Other Compositions and Uses

In another aspect, the present disclosure pertains to a pharmaceutical composition comprising a DVD-Ig molecule or a DVD-Ig DC described herein and a pharmaceutically acceptable excipient or carrier.

In another aspect, the present disclosure pertains to a method of treating prostate cancer, comprising administering to a subject (e.g., a patient) in need thereof, a DVD-Ig drug conjugate described herein, or a pharmaceutical composition thereof.

In another aspect, the present disclosure pertains to nucleic acid molecules encoding a heavy chain and/or a light chain of a DVD-Ig molecule described herein.

In another aspect, the present disclosure pertains to vectors comprising nucleic acid molecules encoding a heavy chain and/or a light chain of a DVD-Ig molecule described herein.

In another aspect, the present disclosure pertains to host cells capable of producing a DVD-Ig molecule described herein.

### EXAMPLES

The following Examples are provided for purposes of illustration, and not limitation.

### Example 1: STEAP1 and PSMA DVD-Ig Generation

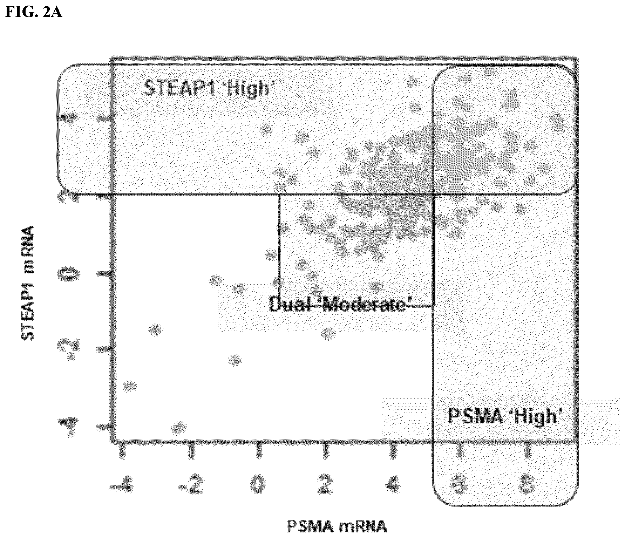

PSMA and STEAP1 are well characterized antigens overexpressed in metastatic prostate cancer with low expression in normal tissue. Both PSMA and STEAP1 are expressed at varying levels in most prostate tumors. To bind both antigens on tumor cells with varied antigen expression, DVD-Igs that target PSMA and STEAP1 were generated. DVD-Ig that targets both antigens can capture a larger patient population, as it can be effective in patients with PSMA1 "high" tumors, STEAP1 "high" tumors, and dual "moderate" expressors (FIG. 2A).

To mitigate risks inherent in the DVD-Ig format, multiple variable regions for PSMA and STEAP1 were identified and humanized for inclusion in a large panel of molecules for screening. The variable regions were derived from mAbs with favorable expression and drug-like property profiles, as any weakness in a variable domain could be amplified when incorporated into a bispecific format. Notably, two well-behaved variable regions could have interactions with each other when placed in a DVD-Ig format that subsequently causes unfavorable properties. Therefore, a library of candidate DVD-Igs were designed and constructed, and a screening was carried out to identify DVD-Igs that have preferred properties.

### Library Design and Plasmid Construction

Parental mAbs for the DVD-Ig molecule were generated using mouse (PSMA) and rat (STEAP1) hybridoma technology and hybridoma supernatants were subsequently screened for activity. Antibody heavy and light chain variable regions (VH and VL) of the selected hybridomas were cloned and expressed as chimeric antibodies, and then further humanized using computer-aided high throughput humanization design software developed internally at AbbVie. Each of the 10 selected humanized PSMA variable regions and each of the 10 selected humanized STEAP1 variable regions were combined with a linker (GS10 on the HC, GS9 on the LC) in both orientations, which resulted in a target library of 200 DVD-Ig molecules.

In transient mammalian expression systems, DVD-Igs were expressed with two plasmids, one for the HC and one for the LC. The plasmids were constructed by overlap extension PCR and ligation. Briefly, each variable domain was amplified by PCR with primers to incorporate an appropriate overhang to the vector or linker. The PCR products were purified and combined for overlap extension PCR. Cleaned up inserts and the pHybE cloning vectors with the constant region were then digested with EcoRI/SalI for the heavy chain (HC) and EcoRI/BsiWI for the light chain (LC). Pooled ligation reactions were transformed into DH5 alpha cells and a 6x oversampling of colonies was screened by colony PCR. For pools with low recovery this step was repeated.

For all DVD-Ig for which matching sequences were identified for both HC and LC plasmids, bacterial cell cultures were re-arrayed into 96-well plates and plasmid DNA was purified for transfection.

### High Throughput Antibody Production (HTAP)

The 5 mL scale HTAP platform was used to express and purify the DVD-Ig in Expi293 cells (Thermo). This production scale generally yields about 100 mg of protein for screening. A Hamilton STAR robot was used to transfect DNA from Costar 3357 96-well plates containing HC and LC DNA in four 24-well plates at a 3:2 ratio with ExpiFectamine^{™} (Gibco A29129). Cultures were fed on day 1. After 5-day transfection, the supernatants containing the DVD-Ig were harvested, re-arrayed and filtered for purification. Protein A purification was done in the 96-well plate format on the Assaymap Bravo. Purified proteins were screened for binding to both PSMA and STEAP1, internalization, and stability by Size Exclusion Chromatography (SEC).

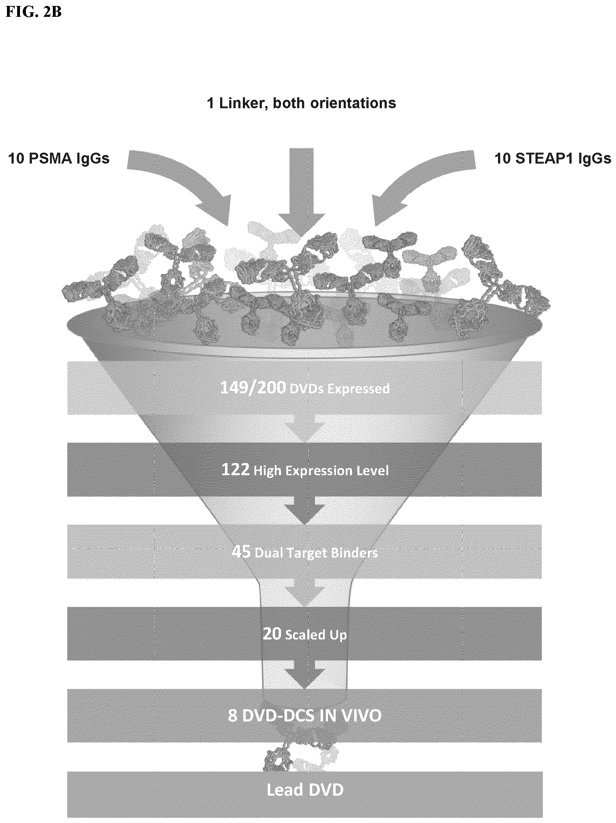

149 of 200 DVD-Igs were successfully expressed, and 122 DVD-Igs had sufficient yield for full characterization. Forty-five dual target binders were identified by FACS binding assay, and 20 were selected for further characterization (FIG. 2B).

### Example 2: Assays and Characterization

The initial drug like properties including the aggregation profiles and the thermal stability of the DVDs shows that a significant percentage of DVD-Igs produced do not meet the aggregation threshold of at least 90% monomer or DSF thermal stability of T-onset >55 °C threshold.

An indirect cytotoxicity assay was used to characterize the HTAP DVD-Igs. LNCaP.FGC cells cultured in RPMI media supplemented with 10% heat-inactivated FBS, and HEK293 cells engineered to express PSMA (HEK293-PSMA) and HEK293 cells engineered to express STEAP1 (HEK293-STEAP1) cultured in DMEM media supplemented with 10% heat-inactivated FBS at mid-log phase were counted (viability, >90%), and 100 µl containing 1500 to 2500 cells were plated into each well of a white 96-well plate (Corning, 3610). After 24 hour incubation at 37°C in a humidified incubator with 5% CO₂, two different dilutions (1:100, 1:1000) of HTAP-generated small scale hybridoma supernatants were incubated with goat anti-human IgG1-MMAF, DAR8 (final concentration 2 µg/mL) conjugated secondary antibody for 20 minutes. Samples were added to each well and incubated for 5 days. Cell viability was assessed with the CellTiter-Glo^{®} Luminescent Cell Viability Assay (Promega, G7570) according to the manufacturer's instructions, with the exception that the plates were shaken for 15 minutes prior to reading. Luminescence was detected on a Perkin Elmer Victor X3 instrument.

Flow cytometry binding assay was also used to characterize the HTAP DVD-Igs. Specifically, HEK293-PSMA or HEK293-STEAP1 cells grown in T-75 flask (Corning, 4430720) were harvested using 5-mL enzyme-free Cell Dissociation Buffer (Invitrogen, 13151-014), washed once with 5 mL FACs buffer (BD Pharmingen, 554657), and then resuspended at 2.5 × 10⁶ cells/mL in FACS buffer. A volume of 100 µL of cells, equivalent to 2.5 ×10⁵ cells/well, was then dispensed into a V-bottom 96-well plate. This was followed by the addition of 100 µL of various concentrations of DVD-Ig, and the samples were incubated at 4°C for 1 hour. Wells were washed twice with FACs buffer and then resuspended in 100 µL of 1:100 anti-human IgG antibody (PE, Southern Biotech, 2040-09) along with Far Red Live/Dead Stain at 1µL/mL (Invitrogen, L34974A) diluted in FACs buffer. The plate was incubated at 4°C for another 1 hour and then washed twice with FACs buffer. Cells were then resuspended in 100 µL of D-PBS (Sigma, D8537)/1% formaldehyde and analyzed on a BD FACS Canto II flow cytometer. The data collected from the cytometer was subsequently analyzed using the FlowJo flow cytometry analysis software.

20 DVD-Igs passed initial production and binding screens. Table 3 provides a summary *of in vitro* potency, flow cytometry binding of DVD-Igs to LNCaP.FGC, and HEK293 cells engineered to express PSMA and STEAP1.

**Table 3**

| **Clone name** | **Outer Domain** | **STEAP FACS Binding GMF** | **PSMA FACS Binding GMF** | **Killing Data (1:100), % Inhib, LnCaP** | **Killing Data (1:1000), % Inhib, LnCaP** | **Killing Data (1:100), % Inhib, 293-PSMA** | **Killing Data (1:1000), % Inhib, 293-PSMA** | **Killing Data (1:100), % Inhib, 293-STEAP1** | **Killing Data (1:1000), % Inhib, 293-STEAP1** |
|---|---|---|---|---|---|---|---|---|---|
| **Clone 1** | **PSMA** | **58.9** | **43.8** | **93.1** | **91.7** | **87.5** | **89.20** | **77.4** | **80.4** |
| **Clone 2** | **PSMA** | **56.2** | **74.9** | **94.1** | **93.0** | **85.7** | **91.23** | **80.5** | **75.7** |
| **Clone 3** | **PSMA** | **39.6** | **50.0** | **91.8** | **92.8** | **88.4** | **89.71** | **79.0** | **72.9** |
| **Clone 4** | **PSMA** | **25.8** | **29.8** | **92.8** | **93.1** | **89.7** | **90.72** | **78.0** | **72.9** |
| **Clone 5** | **PSMA** | **53.0** | **49.5** | **93.5** | **93.1** | **84.6** | **90.43** | **80.5** | **71.9** |
| **Clone 6** | **PSMA** | **19.0** | **26.5** | **92.2** | **90.4** | **88.3** | **87.96** | **76.9** | **71.6** |
| **Clone 7** | **PSMA** | **24.2** | **20.4** | **93.0** | **92.7** | **90.8** | **92.64** | **79.2** | **69.0** |
| **Clone 8** | **PSMA** | **64.3** | **44.7** | **92.7** | **92.5** | **87.4** | **90.26** | **77.0** | **66.9** |
| **Clone 9** | **PSMA** | **6.4** | **10.5** | **90.0** | **93.3** | **91.2** | **91.67** | **76.2** | **64.7** |
| **Clone 10** | **PSMA** | **26.1** | **49.3** | **93.4** | **93.1** | **89.4** | **90.63** | **78.8** | **57.5** |
| **Clone 11** | **STEAP** | **7.8** | **3.5** | **92.4** | **92.6** | **85.7** | **81.2** | **83.8** | **83.2** |
| **Clone 12** | **STEAP** | **37.7** | **10.9** | **91.9** | **93.3** | **89.1** | **80.7** | **81.9** | **84.3** |
| **Clone 13** | **STEAP** | **58.6** | **18.7** | **92.6** | **93.8** | **88.3** | **78.5** | **85.3** | **84.8** |
| **Clone 14** | **STEAP** | **34.3** | **5.0** | **91.3** | **92.2** | **87.2** | **76.9** | **83.1** | **86.4** |
| **Clone 15** | **STEAP** | **102.7** | **25.8** | **91.2** | **92.3** | **86.3** | **74.9** | **80.2** | **81.3** |
| **Clone 16** | **STEAP** | **74.7** | **21.7** | **93.6** | **93.2** | **83.8** | **71.9** | **84.3** | **85.9** |
| **Clone 17** | **STEAP** | **17.9** | **3.2** | **93.1** | **93.9** | **81.9** | **70.4** | **80.6** | **84.4** |
| **Clone 18** | **STEAP** | **68.5** | **24.9** | **93.4** | **93.5** | **85.6** | **68.3** | **84.6** | **84.8** |
| **Clone 19** | **STEAP** | **80.0** | **16.0** | **92.7** | **92.7** | **85.1** | **61.4** | **84.9** | **85.3** |
| **Clone 20** | **STEAP** | **59.2** | **14.5** | **92.3** | **91.4** | **81.9** | **60.3** | **79.1** | **83.8** |

### Example 3: MMAE-conjugated PSMA-STEAP1 DVD-Igs Demonstrated Cytotoxic Activity in vitro

Eight PSMA-STEAP1 DVD-Igs (Clone 2, Clone 4, Clone 9, Clone 11, Clone 12, Clone 13, Clone 14, and Clone 15) were conjugated with monomethyl auristatin E (MMAE) and evaluated in LNCaP.FGC, HEK293-STEAP1, and HEK293-PSMA cells *in vitro.* An anti-STEAP1-MMAE ADC and two comparable anti-PSMA-MMAE ADCs were used as controls.

Cells (LNCaP.FGC, HEK293-PSMA, and HEK293-STEAP1) were counted (viability, >90 %), and 100 µL of medium containing 1500 to 2500 cells were plated into each well of a white 96-well plate (Corning, 3610). After 24 hours incubation at 37°C in a humidified incubator with 5% CO₂, 50 µL of 3x stock solution of conjugate was added per well, and cells were incubated for additional 7days. Samples were analyzed in triplicate. Cell viability was assessed with the CellTiter-Glo^{®} Luminescent Cell Viability Assay (Promega, G7570) according to the manufacturer's instructions, with the exception that the plates were shaken for 15 minutes prior to reading. Luminescence was detected on a Perkin Elmer Victor X3 instrument. Untreated control wells were included on each plate. Cells treated with AB095 DVD-Ig, an anti-tetanus toxoid DVD-Ig, conjugated with MMAE was also included as a control.

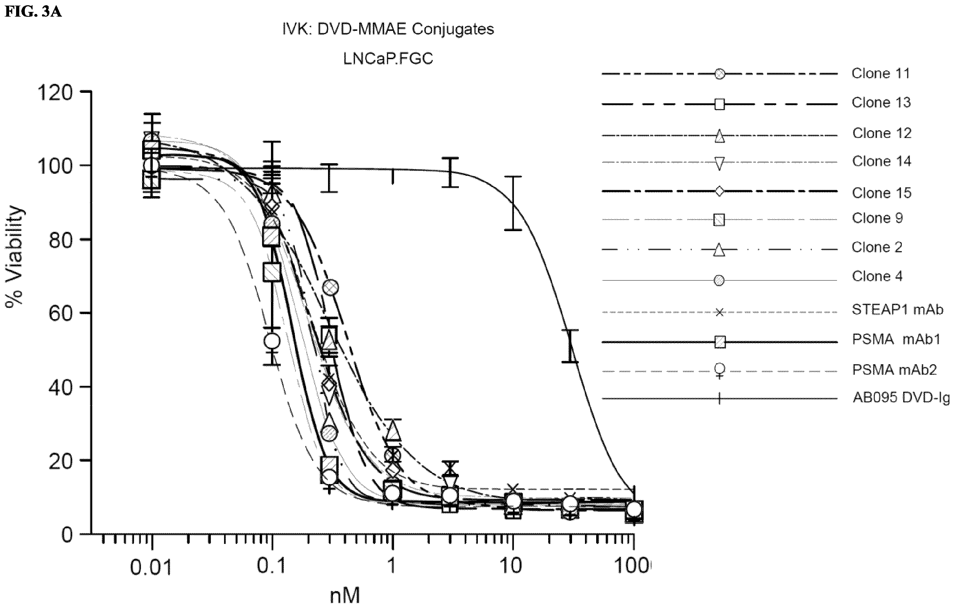

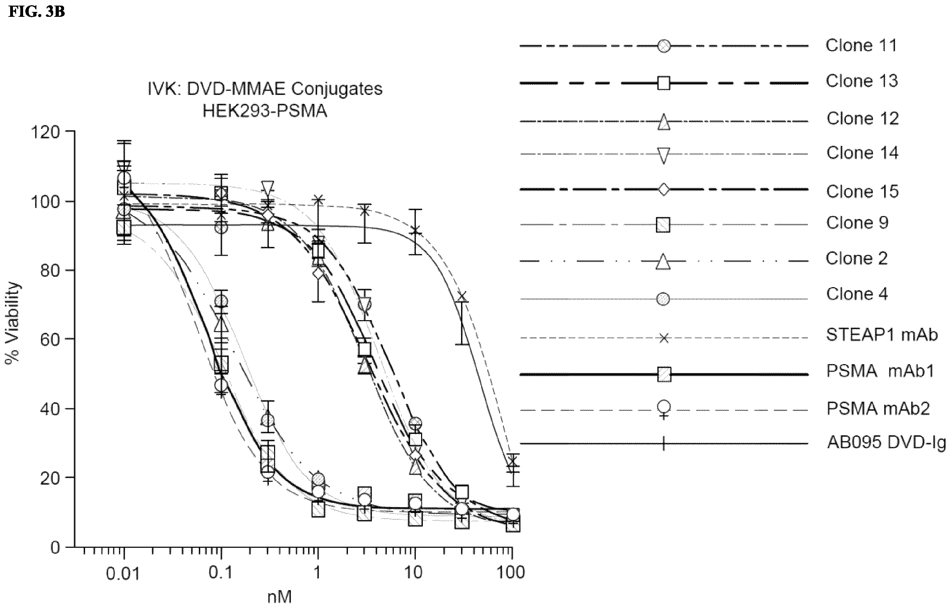

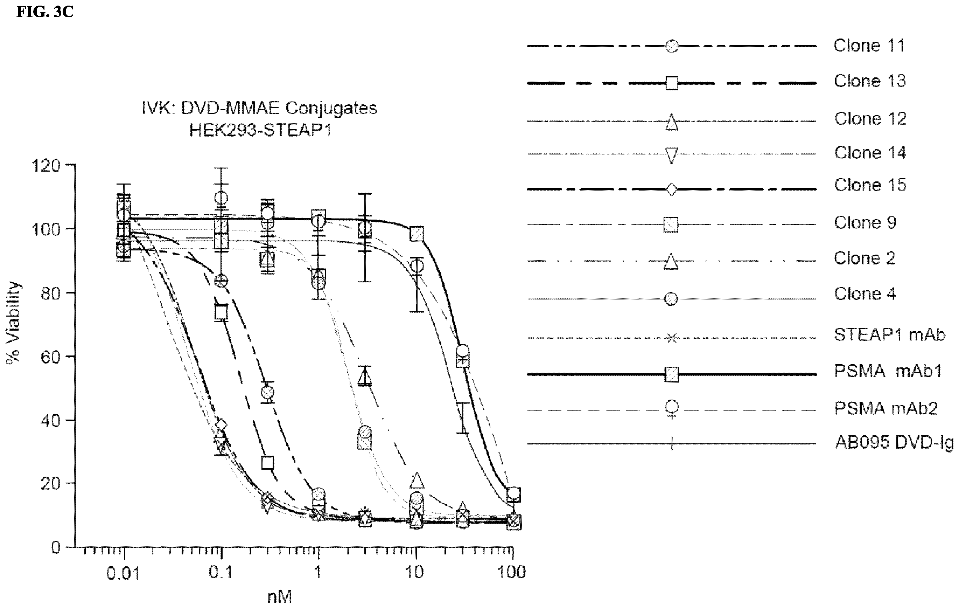

All eight PSMA-STEAP1 DVD-Igs conjugated with MMAE showed cytotoxic activity in LNCaP.FGC cells (FIG. 3A), HEK293-PSMA (FIG. 3B), and HEK-STEAP1 (FIG. 3C) *in vitro.* Their IC50 were shown in Table 5A below.

**Table 5A. In vitro cytotoxic activity of eight DVD-Ig MMAE conjugates**

| | Clone 11 | Clone 13 | Clone 12 | Clone 14 | Clone 15 | Clone 9 | Clone 2 | Clone 4 | STEAP 1 | PSM A | PSM A | Ab09 5 |
|---|---|---|---|---|---|---|---|---|---|---|---|---|
| Outer domain | STEAP 1 | STEAP 1 | STEAP 1 | STEAP 1 | STEAP 1 | PSM A | PSM A | PSM A | | | | |
| LNCaP.FGC IC50 (rM) | 0.42 | 0.31 | 0.29 | 0.20 | 0.22 | 0.13 | 0.22 | 0.17 | 0.22 | 0.15 | 0.10 | 30.02 |
| HEK293-PSMA IC50 (rM) | 5.58 | 3.78 | 3.04 | 4.21 | 3.27 | 0.10 | 0.14 | 0.17 | 104.10 | 0.07 | 0.06 | 44.53 |
| HEK-STEAP1 IC50 (rM) | 0.29 | 0.16 | 0.05 | 0.04 | 0.06 | 2.17 | 3.09 | 2.00 | 0.03 | 30.64 | 80.42 | 23.10 |

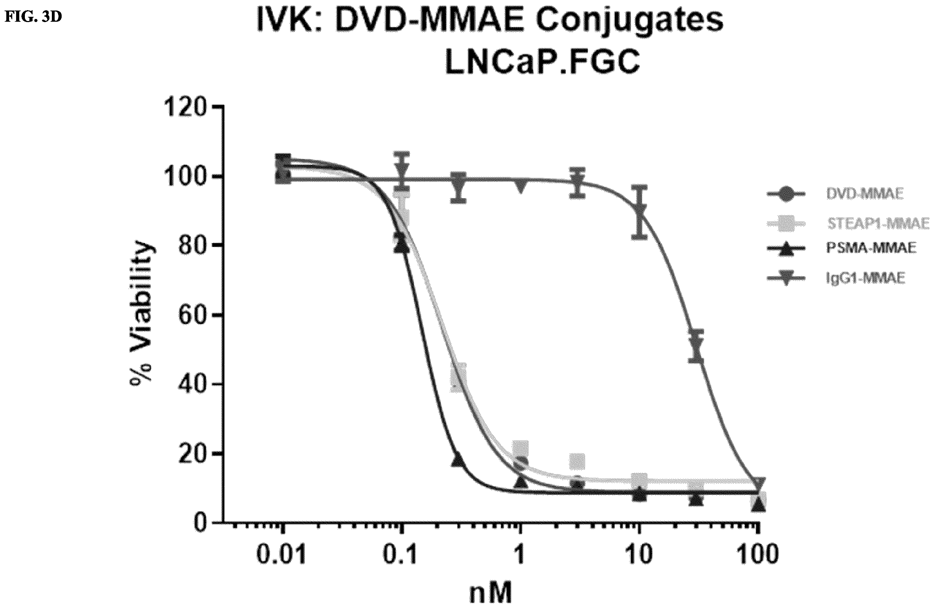

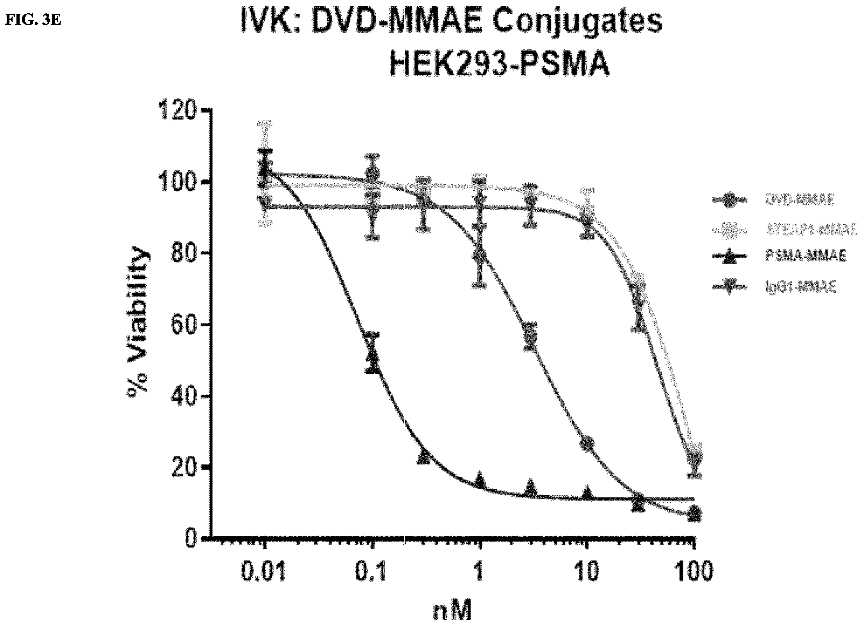

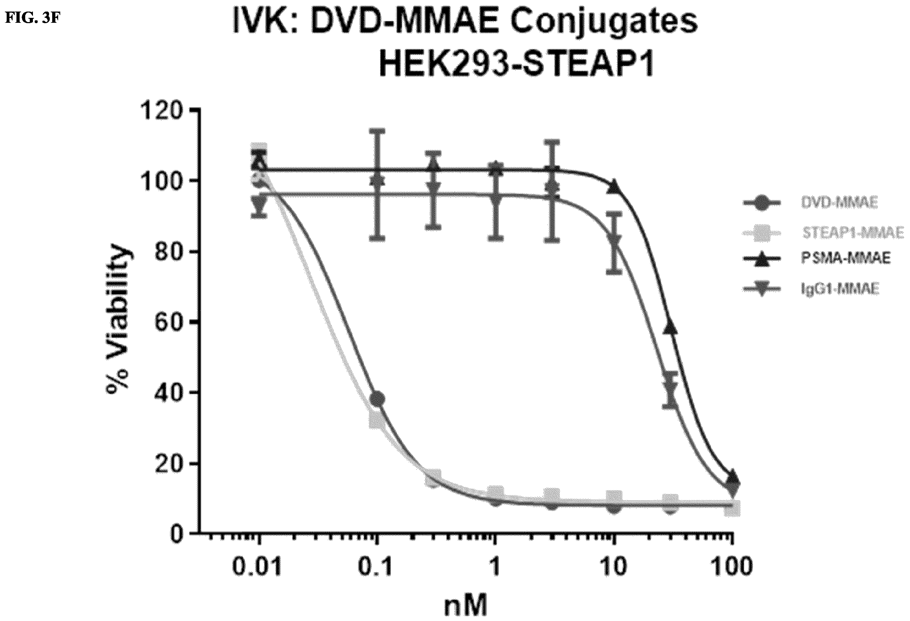

*In vitro* cytotoxic activity of PSMA-STEAP1 DVD-Ig clone 15 conjugated with MMAE ("DVD-MMAE") was confirmed and shown in FIGS. 3D-3F. FIG. 3D shows that DVD-MMAE activity was similar to PSMA-MMAE or STEAP1-MMAE in LNCaP.FGC cells expressing both targets. FIG. 3E shows that DVD-MMAE activity was weaker than PSMA-MMAE in HEK293-PSMA cells. This is expected due to PSMA being the inner variable domain of DVD-Ig. FIG. 3F shows that DVD-MMAE activity was similar to STEAP1-MMAE in HEK293-STEAP1 cells.

### Example 4: MMAE-conjugated PSMA-STEAP1 DVD-Igs Demonstrated Cytotoxic Activity in Xenograft Models

Seven of the eight MMAE-conjugated PSMA-STEAP1 DVD-Igs (Clone 4, Clone 9, Clone 11, Clone 12, Clone 13, Clone 14, and Clone 15) that showed *in vitro* cytotoxic activity were further evaluated in *in vivo* studies. MMAE-conjugated PSMA-STEAP1 DVD-Ig Clone 2 was not included in the *in vivo* studies due to it having a high percentage of aggregation.

Male NSG mice were obtained from Jackson Laboratories (Bar Harbor, ME) for both LNCaP.FGC and 22Rv1 xenograft studies. Mice were housed by treatment group at seven to eight mice per cage. Food and water were available ad libitum. Mice were acclimated to the animal facilities for a period of at least one week prior to commencement of experiments. Animals were tested in the light phase of a 12-hour light: 12-hour dark schedule (lights on at 06:00 hours).

To generate xenografts from LNCaP.FGC (ATCC) and from 22Rv1 (ATCC) cell lines, 5 × 10⁶ viable LNCaP.FGC and 22Rv1 cells were inoculated subcutaneously into the right flank of male NSG mice, respectively. The injection volume was 0.1 mL and composed of a 1:1 mixture with Matrigel (BD, Franklin Lakes, NJ). Measurements of the length (L) and width (W) of the tumor were taken via electronic caliper and the tumor volume was calculated according to the following equation: V = L × W²/2.

Mice were assigned to treatment groups so that the mean tumor volumes within groups were approximately 175-250 mm³. An anti-PSMA-MMAE ADC at Dose-1 1X, an anti-STEAP1-MMAE ADC at Dose-1 1X, and various of the PSMA/STEAP-1 DVD-Igs conjugated with MMAE described above at Dose-1 1.33X (Clone 13 was administered at Dose-1 0.87X due to limited compound availability) were administered within 24-48 hours of assignment to treatment groups based on tumor volumes. Tumor volumes were estimated two times weekly after dosing, with study groups body weights taken at the time of tumor measurements. Mice were euthanized (1) when tumor volume reached 2,000 mm³, (2) when general efficacy parameters were confidently determined, or (3) when necessitated by other animal health issues.

Parameters of Efficacy were determined as follows:
TGI (tumor growth inhibition) = 1 - (mean tumor volume of treatment group/mean tumor volume of treatment control group) × 100 based on measurements on day of vehicle endpoint. P values are derived from Student's T test comparison of treatment group vs. vehicle control group.

TGD (tumor growth delay) = (T - C)/C × 100, where T is the median time to endpoint of treatment group and C is the median time to endpoint of treatment control group. P values are derived from Kaplan Meier log-rank comparison of treatment group vs. vehicle control group.

The parameters of efficacy for all 7 PSMA-STEAP1 DVD-Ig clones conjugated with MMAE tested in the LNCaP.FGC xenograft studies are shown in Table 5B below.

**Table 5B**

| **LNCaP.FGC (ATCC)** | | | | | | | |
|---|---|---|---|---|---|---|---|
| TGI (%) day of comparison: d63 post inoculation | | | | TGD (%) tumor volume endp oint: 1000 mm³ | | | |
| | | | **Parameters of Efficacy** | | | | |
| **Compound(s)** | **Dose (mg/kg)** | **Regimen** | **TGI (%)** | **TGD (%)** | **CR (%)** | **PR (%)** | **TR (%)** |
| PSMA-(vcMMAE) (DAR4) | Dose-1 1X | IP, QD x 1 | 92*** | 202*** | 0 | 100*** | 100*** |
| STEAP1-(vcMMAE) (DAR4) | Dose-1 1X | IP, QD x 1 | 69** | 114** | 13 | 0 | 13 |
| STEAP1-PSMA-vcMMAE Clone 11 | Dose-1 1.33X | IP, QD x 1 | 35 | 53 | 0 | 0 | 0 |
| STEAP 1 -PSMA-vcMMAE Clone 13 | Dose-1 0.87X | IP, QD x 1 | 33 | 53 | 0 | 0 | 0 |
| STEAP1-PSMA-vcMMAE Clone 12 | Dose-1 1.33X | IP, QD x 1 | 40 | 59* | 0 | 0 | 0 |
| STEAP 1-PSMA-vcMMAE Clone 14 | Dose-1 1.33X | IP, QD x 1 | 64 | 86*** | 0 | 0 | 0 |
| STEAP1-PSMA-(vcMMAE) Clone 15 | Dose-1 1.33X | IP, QD x 1 | 88*** | 229*** | 38 | 38* | 75*** |
| PSMA-STEAPl-vcMMAE Clone 9 | Dose-1 1.33X | IP, QD x 1 | 69 | 114*** | 0 | 63* | 63* |
| PSMA-STEAP 1 - vcMMAE Clone 4 | Dose-1 1.33X | IP, QD x 1 | 67 | 86*** | 0 | 38 | 38 |
| Vehicle | - | IP, QD x 1 | NA | NA | 0 | 0 | 0 |

| | | | | | | | |
|---|---|---|---|---|---|---|---|
| CR (complete response) is the percentage of mice within a group with a tumor burden ≤ 25 mm³ for at least three consecutive measurements. PR (partial response) is the percentage of mice within a group with a tumor burden larger than 25 mm³ but less than one-half of the volume at onset of treatment for at least three consecutive measurements. TR (total responders) is the sum of CR and PR. P values for these response rates are derived from the Fisher's Exact Test for Response Frequency for comparison of treatment group to treatment control group. *** = p < 0.001 when compared to the control group; ** = p < 0.01 when compared to the control group; * = p < 0.05 when compared to the control group. | | | | | | | |

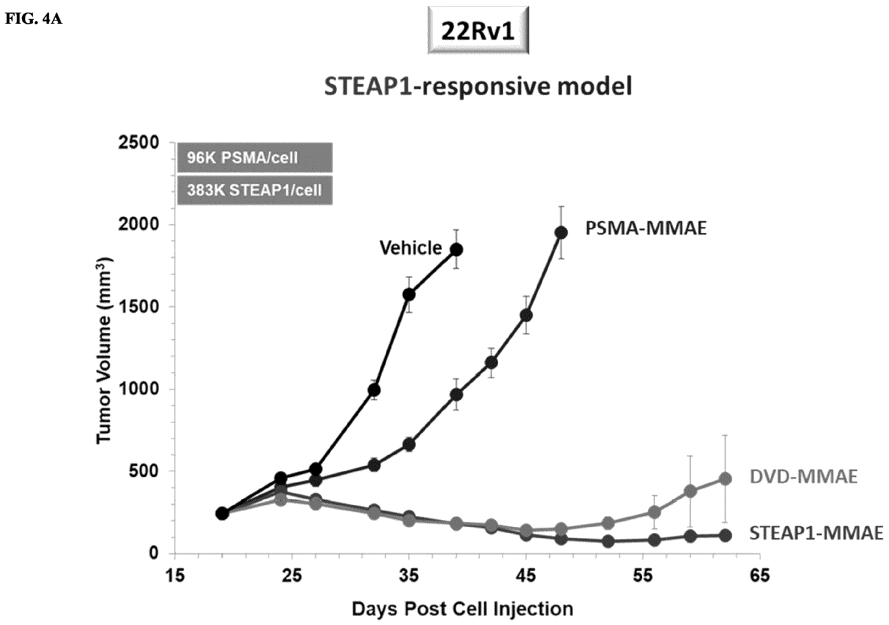

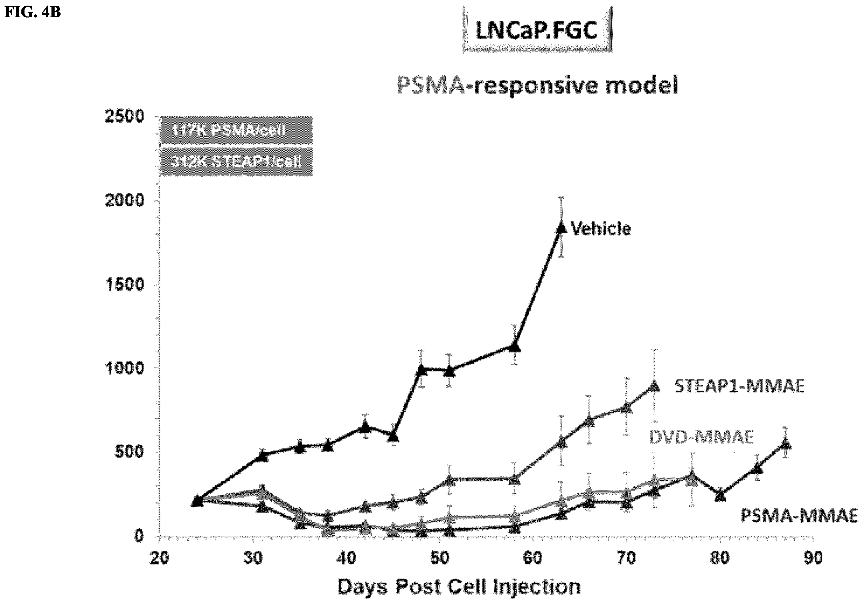

PSMA-STEAP1 DVD-Ig clone 15 conjugated with MMAE ("DVD-MMAE") showed comparable or superior activity relative to monospecific ADCs ("PSMA-MMAE" and "STEAP1-MMAE") in 22Rv1 (FIG. 4A) and LNCaP.FGC (FIG. 4B) xenograft models. Surface expression of PSMA and STEAP1 is high in both 22Rv1 and LNCaP.FGC models.

### Example 5: Liability Engineering of The Lead Candidate

Based on the results of the *in vitro* screening and the *in vivo* study, clone 15 was selected for further evaluation and engineering. It demonstrated good potency and acceptable drug-like properties, but manifested a deamidation motif (20%) in the PSMA binding domain.

Rational design was performed to identify and remove high risk sequence liabilities. DVDs with mutations were produced and evaluated in a Biacore assay, where the kinetics of binding to PSMA resulted in the identification NT as a non-disruptive mutation away from "NG". NT was used in this position of HCDR2 in further versions of the DVD-Ig since no significant loss of binding occurred. In order to facilitate DAR2 or DAR6 conjugation, the variable regions were cloned onto a light chain containing a Cysteine to Alanine mutation. This creates an unpaired cysteine at position C220 of the HC for conjugation of the linker drug in a facile, site-specific manner.

DVD-Ig1 was generated by performing deamidation of VH2-CDR2 of clone 15, and the sequence is shown below in Table 1.

**Table 1.**

| **Protein Region** | **V Region** | **SEQ ID NO** |
|---|---|---|
| Heavy Chain_vl | EVQLVESGGGVVQPGRSLRLSCVASGFPFNNYWMTWIRQ APGKGLEWIASITITGGTTYYPGSVKGRFTISRDNSKSTLYL QMNSLRAEDTAVYYCTRERYSTEYYPYYWYFDFWGQGT MVTVSSGGGGSGGGGSEVQLVQSGAEVKKPGSSVKVSCKA SGYTFTDYYMNWVKQAPGKGLEWIGLINPSNTGTNYNQK FRDKATLTVDKSTSTAYMELSSLRSEDTAVYYCARRAQAT SGDYWGQGTTVTVSSASTKGPSVFPLAPSSKSTSGGTAALGC LVKDYFPEPVTVSWNSGALTSGVHTFPAVLQSSGLYSLSSVVT VPSSSLGTQTYICNVNHKPSNTKVDKKVEPKSCDKTHTCPPCP APEAAGGPSVFLFPPKPKDTLMISRTPEVTCVVVDVSHEDPEV KFNWYVDGVEVHNAKTKPREEQYNSTYRVVSVLTVLHQDW LNGKEYKCKVSNKALPAPIEKTISKAKGQPREPQVYTLPPSRE EMTKNQVSLTCLVKGFYPSDIAVEWESNGQPENNYKTTPPVL DSDGSFFLYSKLTVDKSRWQQGNVFSCSVMHEALHNHYTQK SLSLSPGK | 1 |
| First Heavy Chain Variable Domain (VH1) | EVQLVESGGGVVQPGRSLRLSCVASGFPFNNYWMTWIRQ APGKGLEWIASITITGGTTYYPGSVKGRFTISRDNSKSTLYL QMNSLRAEDTAVYYCTRERYSTEYYPYYWYFDFWGQGT MVTVSS | 2 |
| VH1-CDR1 | **GFPFNNYWMT** | 3 |
| VH1-CDR2 | **SITITGGTTYYPGSVKG** | 4 |
| VH1-CDR3 | **ERYSTEYYPYYWYFDF** | 5 |
| Linker | ***GGGGSGGGGS*** | 6 |
| Second Heavy Chain Variable Domain (VH2) | EVQLVQSGAEVKKPGSSVKVSCKASGYTFTDYYMNWVKQ APGKGLEWIGLINPSNTGTNYNQKFRDKATLTVDKSTSTA YMELSSLRSEDTAVYYCARRAQATSGDYWGQGTTVTVSS | 7 |
| VH2-CDR1 | **GYTFTDYYMN** | 8 |
| VH2-CDR2 | **LINPSNTGTNYNQKFRD** | 9 |
| VH2-CDR3 | **RAQATSGDY** | 10 |
| Light Chain | DIQMTQSPSSLSASVGDRVTITCRTSQDIGNYLRWFQQKPG KSPKLMIYDAINLAAGVPSRFSGSGSGTDYTLTISSLQPEDV ATYYCLQHNEYPYTFGQGTKLEIKRGGSGGGGSGDIQMTQ SPSSLSASVGDRVTITCHASQNINVWLSWYQQKPGKAPKL LIYKASNLHTGVPSRFSGSGSGTDFTFTISSLQPEDIATYYC QQGQTYPLTFGGGTKVEIKRTVAAPSVFIFPPSDEQLKSGTA SVVCLLNNFYPREAKVQWKVDNALQSGNSQESVTEQDSKDS TYSLSSTLTLSKADYEKHKVYACEVTHQGLSSPVTKSFNRGEA | 11 |
| First Light Chain Variable Domain (VL1) | DIQMTQSPSSLSASVGDRVTITCRTSQDIGNYLRWFQQKPG KSPKLMIYDAINLAAGVPSRFSGSGSGTDYTLTISSLQPEDV ATYYCLQHNEYPYTFGQGTKLEIK | 12 |
| VL1-CDR1 | **RTSQDIGNYLR** | 13 |
| VL1-CDR2 | **DAINLAA** | 14 |
| VL1-CDR3 | **LQHNEYPYT** | 15 |
| Linker | ***GGSGGGGSG*** | 16 |
| Second Light Chain Variable Domain (VL2) | DIQMTQSPSSLSASVGDRVTITCHASQNINVWLSWYQQKP GKAPKLLIYKASNLHTGVPSRFSGSGSGTDFTFTISSLQPED IATYYCQQGQTYPLTFGGGTKVEIK | 17 |
| VL2-CDR1 | **HASQNINVWLS** | 18 |
| VL2-CDR2 | **KASNLHT** | 19 |
| VL2-CDR3 | **QQGQTYPLT** | 20 |
| CH1CH2CH3_v 1 | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPEAAGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSREEMTKNQVSLTCLVKGF YPSDIAVEWESNGQPENNYKTTPPVLDSDGSFFLYSKLTVDKS RWQQGNVFSCSVMHEALHNHYTQKSLSLSPGK | 21 |
| Cκ | RTVAAPSVFIFPPSDEQLKSGTASVVCLLNNFYPREAKVQWKV DNALQSGNSQESVTEQDSKDSTYSLSSTLTLSKADYEKHKVY ACEVTHQGLSSPVTKSFNRGEA | 22 |
| CH1CH2CH3_v 2 | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPEAAGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSREEMTKNQVSLTCLVKGF YPSDIAVEWESNGQPENNYKTTPPVLDSDGSFFLYSKLTVDKS RWQQGNVFSCSVMHEALHNHYTQKSLSLSPG | 23 |
| Heavy Chain_v2 | EVQLVESGGGVVQPGRSLRLSCVASGFPFNNYWMTWIRQ APGKGLEWIASITITGGTTYYPGSVKGRFTISRDNSKSTLYL QMNSLRAEDTAVYYCTRERYSTEYYPYYWYFDFWGQGT MVTVSSGGGGSGGGGSEVQLVQSGAEVKKPGSSVKVSCKA SGYTFTDYYMNWVKQAPGKGLEWIGLINPSNTGTNYNQK FRDKATLTVDKSTSTAYMELSSLRSEDTAVYYCARRAQAT SGDYWGQGTTVTVSSASTKGPSVFPLAPSSKSTSGGTAALGC LVKDYFPEPVTVSWNSGALTSGVHTFPAVLQSSGLYSLSSVVT VPSSSLGTQTYICNVNHKPSNTKVDKKVEPKSCDKTHTCPPCP APEAAGGPSVFLFPPKPKDTLMISRTPEVTCVVVDVSHEDPEV KFNWYVDGVEVHNAKTKPREEQYNSTYRVVSVLTVLHQDW LNGKEYKCKVSNKALPAPIEKTISKAKGQPREPQVYTLPPSRE EMTKNQVSLTCLVKGFYPSDIAVEWESNGQPENNYKTTPPVL DSDGSFFLYSKLTVDKSRWQQGNVFSCSVMHEALHNHYTQK SLSLSPG | 24 |

### Example 6: Generation of DVD-Ig1 Top1i Drug Conjugate

As shown in the examples above, DVD-Ig1 was identified based on expression, drug-like properties, *in vitro* dual binding potency, in vivo activity, and absence of sequence liability. It was then used to generate a Top1i drug conjugate.

The structure of Top1i drug-linker is shown as follows:

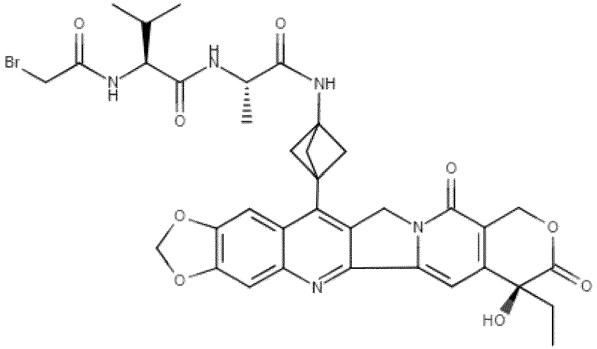

509.9 mL antibody solution (11.34 mg/mL) in PBS was added to 2.0 mL of 0.5 M pH 7.5 EDTA solution. A solution of diphenyl-phosphino-4-benzoic acid (10 mM, 18.38 mL, 2.5 eq) in DMA was then added and the mixture was kept at 4 °C overnight (18 hours) for reduction. 51.6 mL (20% v/v) 100 mM pH 8.5 sodium bicarbonate buffer was added to the reduced antibody, and then treated with the drug-linker solution (10 mM, 11.4 mL, 4.0 equivalence to antibody) and kept at 4 °C for 60 minutes. Crude conjugate was purified to remove excessive drug-linker and other small-molecule reagent.

### HIC analysis:

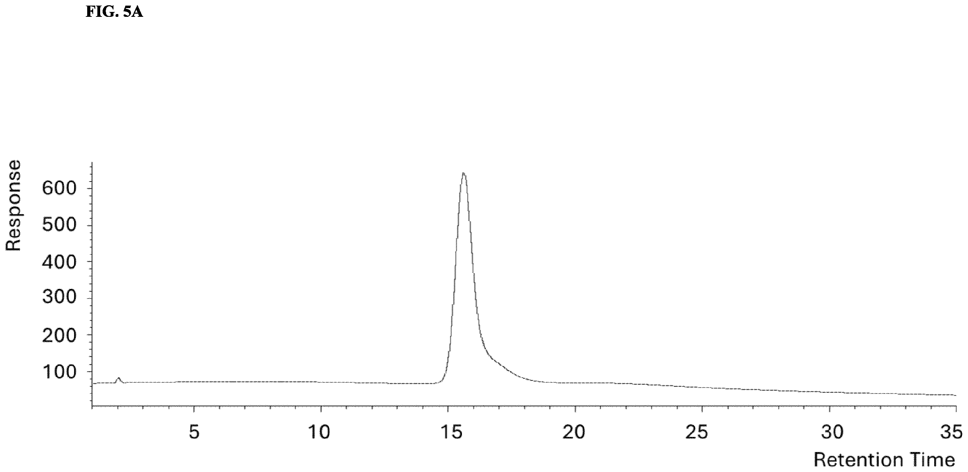

Hydrophobic interaction chromatography was performed using TSK-GEL^{®} Ether-5PW Column (TOSOH Bioscience, 2.0mm ID × 7.5cm, 10 µM) at 30 °C. ADC was 50% Buffer A, loaded and eluted with 10% B for 1 minute, followed by a gradient of 10% B to 100% B in 30 minutes. Mobile Phase A was 25 mM sodium phosphate buffer, 1.5 M ammonium sulfate, pH 7.0; Mobile Phase B was mixture of 75% 25 mM sodium phosphate buffer pH 7.0 and 25% isopropanol, and flow rate was 0.1 mL/min. Data is shown in FIG. 5A.

### Aggregation analysis:

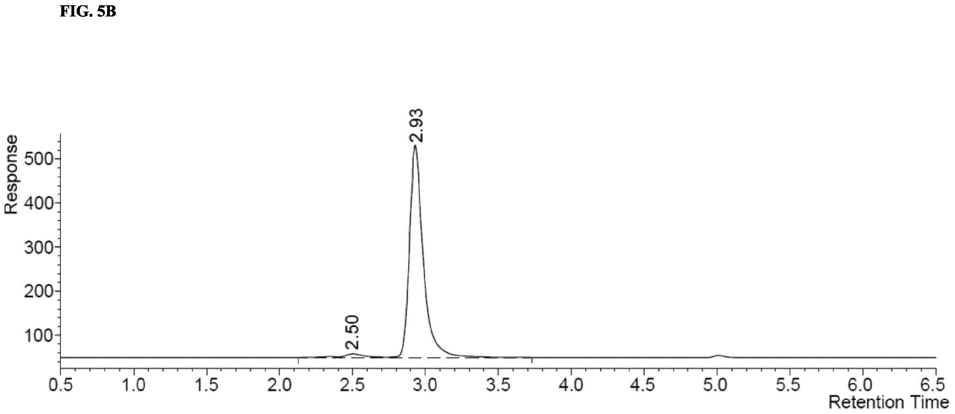

Size exclusion chromatography was performed using Waters ACQUITY UPLC^{®} Protein BEH SEC Column (200 Å, 1.7 µm, 4.6 mm × 150 mm) with 100 mM sodium phosphate, 150 mM sodium chloride, 10% volume/volume acetonitrile, pH 7.0 at a flow rate of 0.4 mL/minute. The peak area absorbance at 280 nm was determined for each of the high molecular weight and monomeric eluents by integration of the area under the curve. The % aggregate fraction of the conjugate sample was determined by dividing the peak area absorbance at 280 nm for the high molecular weight eluent by the sum of the peak area absorbances at 280 mM of the high molecular weight and monomeric eluents multiplied by 100%. Percent aggregation was determined at 2.7% (FIG. 5B).

### Mass Spec analysis:

LC-MS analysis was performed using an Agilent 1100 HPLC system interfaced to an Agilent LC/MSD TOF 6220 ESI mass spectrometer. The ADC was reduced with 25mM (final concentration) Bond-Breaker^{®} TCEP solution (Thermo Scientific, Rockford, IL), loaded onto a Protein Microtrap (Michrom Bioresorces, Auburn, CA) desalting cartridge, and eluted with a gradient of 10% B to 75% B in 0.2 minutes at ambient temperature. Mobile phase A was H2O with 0.1% formic acid, mobile phase B acetonitrile with 0.1% formic acid, and flow rate was 0.2 mL/minute. Electrospray-ionization time-of-flight mass spectra of the co-eluting light and heavy chains were acquired using Agilent MassHunter^{™} acquisition software. The extracted intensity vs. m/z spectrum was deconvoluted using the Maximum Entropy feature of MassHunterp software to determine the mass of each reduced antibody fragment. DAR was calculated from the deconvoluted spectrum by summing intensities of the naked and modified peaks for the light chain and heavy chain, normalized by multiplying intensity by the number of drugs attached. The summed, normalized intensities were divided by the sum of the intensities, and the summing results for two light chains and two heavy chains produced a final average DAR value for the full ADC.

MS data:
35836 Dalton - Light Chain (LC, LC+0)
65144 Dalton - Heavy Chain (HC+0, des-K, G0F)
65827 Dalton - Heavy Chain + 1 Drug (HC+1)

**Table 4**

| | MW | Height |
|---|---|---|
| LC+0 | 35836 | 1099963.0 |
| | | |
| HC+0 | 65144 | 197.2 |
| HC+1 | 65827 | 172150.4 |
| LC Loading | | 0.000 |
| HC Loading | | 0.999 |
| | **DAR** | **2.00** |

### Example 7: Characterization of PSMA-STEAP1-Top1i In Vitro

Internalization and *in vitro* cytotoxicity of DVD-Ig1 drug conjugate was characterized.

### Internalization Assay

Cells were maintained in a humidified 37°C incubator with 5% CO2. Exponetially growing LNCaP.FGC cells were detached using 0.25% trypsin-EDTA (Sigma, T4049) and 20,000 cells/well were plated in 96 well-plates (Corning, 3610) in 50 µl cell culture media. Next day, antibody conjugates (PSMA-STEAP1-Top1i, PSMA-Top1i, and STEAP1-Top1i) and FabFluor reagent (Sartorius, 4722) were mixed at a molar ratio of 1:3 in media, for 15 minutes. The labeled antibodies were serially diluted and added to each well in triplicates. The plates were placed in Incucyte Zoom (Sartorius) for image collection for up to 24 hours. Collected images were processed and analyzed. The final data was exported to the GraphPad Prism software for graph generation.

### Intracellular Accumulation Assay

The intracellular accumulation assay was used to measure mean intracellular concentrations of unconjugated Top1i. Specifically, 2 million LNCaP.FGC cells in 40 ml medium were plated in a T150 flask 24 hours prior to the treatment. Each T150 flask was treated with 0 nM, 1 nM, 10 nM, or 30 nM of PSMA-STEAP1-Top1i, PSMA-Top1i, or STEAP1-Top1i. Samples were collected at timepoints 4, 6, and 24 hours. Two technical replicates at each time point were used. Cells without treatment (0 nM) was the control. At each time point, cells were washed twice with PBS and detached with trypsin to prepare single cell suspension. Live cell count and cell diameter were determined from the single cell suspension to calculate total cellular volume (in liter). LC-MS analysis was conducted to determine the amount of intracellular small molecule (in nanomoles). The LC-MS method quantified total cell associated molecules which were not covalently bound to any entity, i.e., sum of bound and unbound molecules in the cell. All molecules covalently bound to cellular proteins/lipids were not extracted and measured. Top1i compound has two forms: the active closed ring (Lactone) form at low pH, or inactive open ring (Carboxylate) form. Protein was precipitated with acidic dilution when preparing samples for LC-MS analysis, because lower pH pushed equilibrium to active closed ring form. During assay, media pH became acidic. The LC-MS bioanalysis converted majority of unconjugated payload to the closed ring status, and hence measured "total payload" (i.e., open form and closed form) irrespective of their original forms present in the sample.

Mean intracellular concentration (in nanomolar) was then calculated based on the total cellular volume and the amount of intracellular small molecule. Additional calculation details are shown below:
$Nanomoles per sample = LCMS concentration of cell sample \left[nM\right] \times Sample volume \left[L\right]$
$Mean Intracellular Drug Concentration \left[nM\right] = \frac{Mean cell associated drug per live cell \left(nanomoles\right)}{Mean single live cell volume = \frac{4}{3} ∗ π ∗ {r}^{3} \left(liters\right)}$
$Number of Molecules per sample = Nanomoles per sample \times {10}^{- 9} \frac{mole}{nanomoles} \times 6.022 \times {10}^{23} \frac{molecules}{mole}$
$Number of Molecules per live cell = \frac{Number of Molecules per sample}{Number of Live Cells per sample}$

### Anti-proliferation assay

LNCaP.FGC cells were dissociated using 0.25% trypsin-EDTA (Sigma, T4049) and counted (viability >90%). 100 mL of growth medium containing 2500 cells were plated into each well of a white 96-well plate (Corning, 3610). After 24-hour incubation at 37°C in a humidified incubator with 5% CO₂, 50 mL of 3× stock solution of conjugate or payload were added per well, and cells were incubated for an additional 7 days. Samples were analyzed in triplicate. Cell viability was assessed with the CellTiter-Glo Luminescent Cell Viability Assay (Promega, G7570) according to the manufacturer's instructions, with the exception that the plates were shaken for 15 minutes prior to reading. Luminescence was detected on a Perkin Elmer Victor X3 instrument. Untreated control wells were included on each plate. Percent of control was calculated using the following formula: 100 × (treated)/(control). Half-maximal inhibition (IC₅₀) values were calculated using GraphPad Prism software.

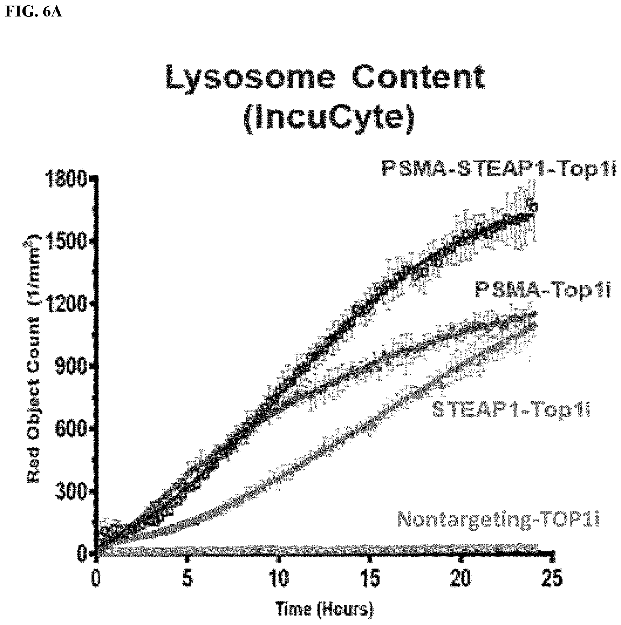

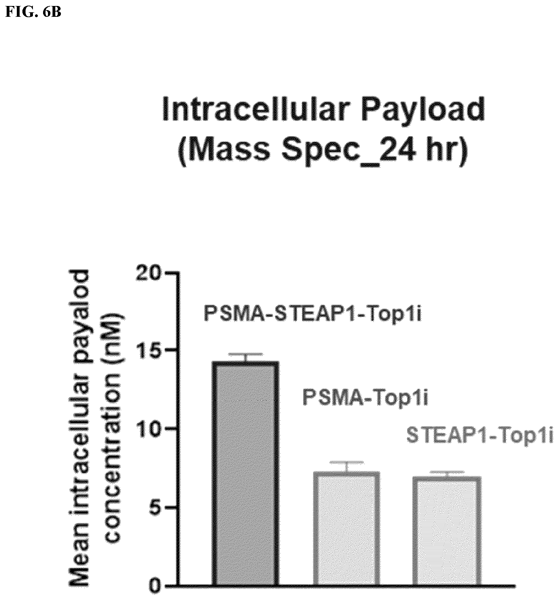

FIGS. 6A and 6B show that DVD-Ig1 conjugated with Top1i at DAR2 ("PSMA-STEAP1-Top1i") internalized faster and delivered more payload compared to either monospecific ADC ("PSMA-Top1i" or "STEAP1-Top1i").

FIG. 6B shows that intracellular payload accumulation was roughly two times higher for PSMA-STEAP1-Top1i than that of either monospecific ADC, PSMA-Top1i or STEAP-Top1i, in cells treated with 30nM drug conjugates at 24 hours.

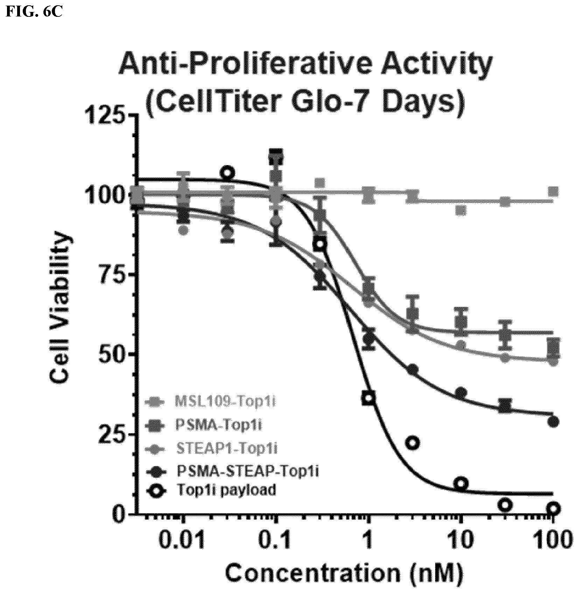

PSMA-STEAP1-Top1i also showed superior anti-proliferative activity than either monospecific ADC, PSMA-Top1i or STEAP-Top1i (FIG. 6C).

### Example 8: Characterization of PSMA-STEAP1-Top1i In Vivo

DVD-Ig1 conjugated with Top1i ("PSMA-STEAP1-Top1i") showed efficacy in prostate tumors that express STEAP1 and PSMA in xenograft models *in vivo.*

Male NSG mice were obtained from Jackson Laboratories (Bar Harbor, ME) for the LNCaP.FGC xenograft study. Male CB17.SCID (Charles River Laboratories, Wilmington, MA) mice were used for the 22Rv1 xenograft study. Mice were housed by treatment group at seven to eight mice per cage. Food and water were available ad libitum. Mice were acclimated to the animal facilities for a period of at least one week prior to commencement of experiments. Animals were tested in the light phase of a 12-hour light: 12-hour dark schedule (lights on at 06:00 hours).

To generate xenografts from LNCaP.FGC (ATCC) and from 22Rv1 (ATCC) cell lines, 5 × 10⁶ viable LNCaP.FGC and 22Rv1 cells were inoculated subcutaneously into the right flank of male NSG mice and male CB17.SCID mice, respectively. The injection volume was 0.1 mL and composed of a 1:1 mixture with Matrigel (BD, Franklin Lakes, NJ). Measurements of the length (L) and width (W) of the tumor were taken via electronic caliper and the tumor volume was calculated according to the following equation: V = L × W²/2.

Mice were assigned to treatment groups so that the mean tumor volumes within groups were approximately 175-250 mm³. For the LNCaP.FGC xenograft study, PSMA-Top1i at Dose-2 (1X), STEAP1-Topli at Dose-2 (1X), and PSMA-STEAP1-Top1i at Dose-2 (1.3X) were administered within 24-48 hours of reassignment. For the LNCaP.FGC xenograft study, PSMA-Top1i at Dose-3 (1X), STEAP1-Top1i at Dose-3 (1X), and PSMA-STEAP1-Top1i at Dose-3 (1.3X) were administered within 24-48 hours of reassignment. Tumor volumes were estimated two times weekly after dosing, with study groups body weights taken at the time of tumor measurements. Mice were euthanized (1) when tumor volume reached 2,000 mm³, (2) when general efficacy parameters were confidently determined or (3) when other animal health issues were observed.

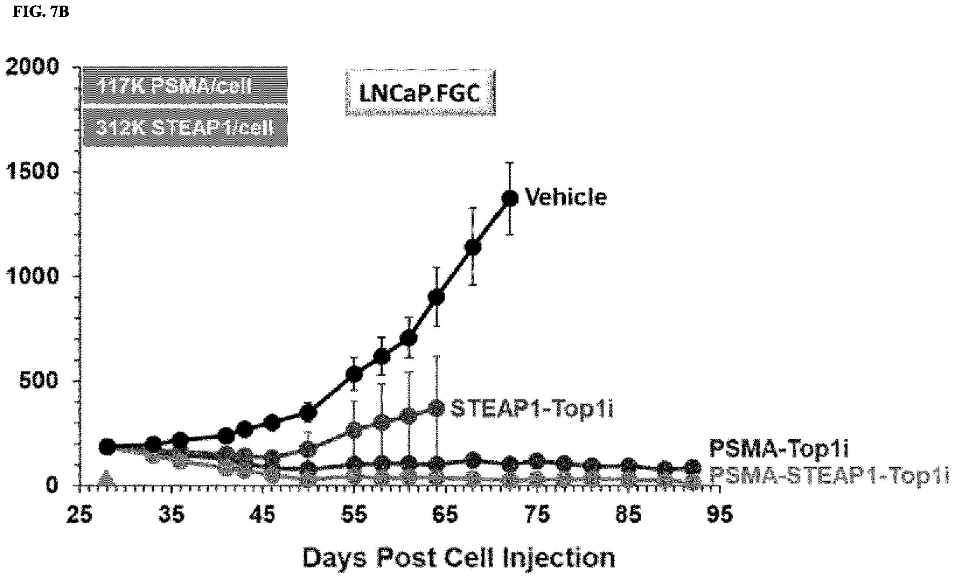

PSMA-STEAP1-Top1i was highly efficacious against LNCaP.FGC subcutaneous flank tumors when administered as a single dose at Dose-2 (1.3X) (FIG. 7B). Treatment against these cell-derived xenografts (CDX) resulted in tumor volume regression over an extended period (>30 - > 100 days) in this model that, by RNA expression analysis, expressed high levels of both PSMA and STEAP1 surface molecules.

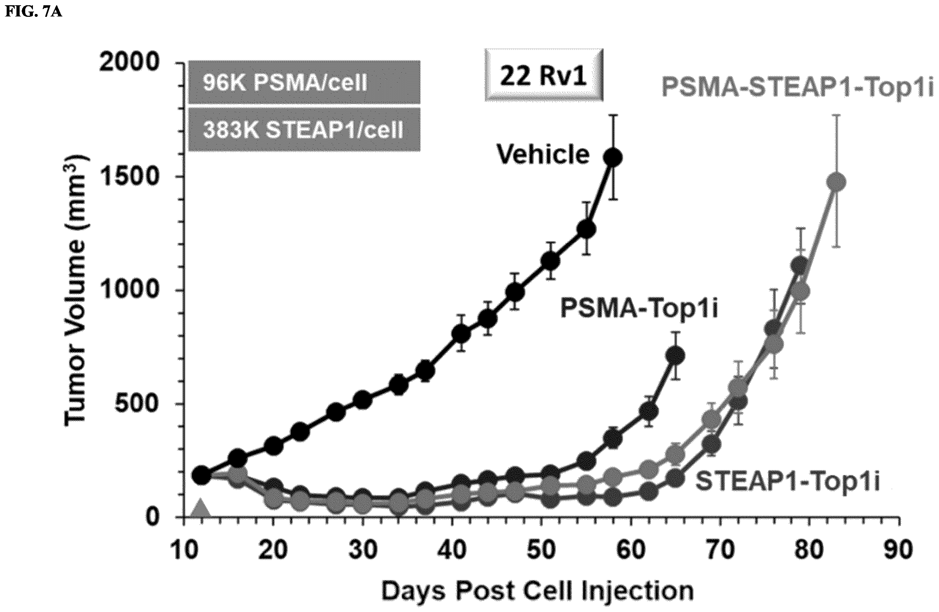

For 22Rv1, a CDX model which also expressed high levels of both PSMA and STEAP1 based on RNA expression, PSMA-STEAP1-Top1i was also highly efficacious at Dose-3 (1.3X) (FIG. 7A). Drug conjugates which target either PSMA ("PSMA-Top1i") or STEAP1 ("STEAP1-Top1i") were similarly efficacious, with differences only seen well beyond single dose administration.

These data indicate that DVD-Ig1 disclosed herein has activity comparable or better than that of individual ADCs, and that a single dose of PSMA-STEAP1-Top1i supports sustained tumor regression.

### Exemplary Embodiments:

Embodiment 1. A dual-variable domain immunoglobulin (DVD-Ig) drug conjugate comprising the following structure: 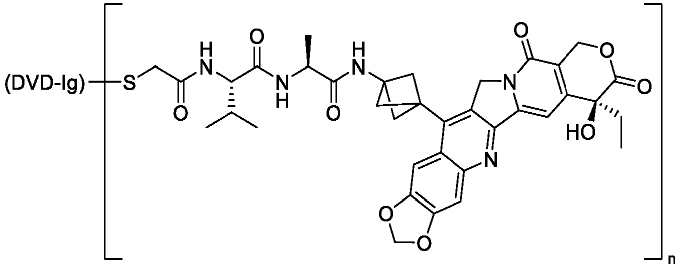
wherein n is 1 or 2,
wherein the DVD-Ig binds human STEAP1 and human PSMA, and comprises two identical heavy chains and two identical light chains,
wherein each heavy chain comprises:
   a) a first heavy chain variable domain (VH1) comprising VH1-CDR1 (SEQ ID NO: 3), VH1-CDR2 (SEQ ID NO: 4), and VH1-CDR3 (SEQ ID NO: 5);
   b) a second heavy chain variable domain (VH2) a comprising VH2-CDR1 (SEQ ID NO: 8), VH2-CDR2 (SEQ ID NO: 9), and VH2-CDR3 (SEQ ID NO: 10); and
   c) an IgG1 heavy chain constant region (CH1CH2CH3);
wherein the VH1 and the VH2 are connected via a first peptide linker (L1), and each heavy chain is in the form of VH1-L1-VH2-CH1CH2CH3;
wherein each light chain comprises:
   a) a first light chain variable domain (VL1) comprising VL1-CDR1 (SEQ ID NO: 13), VL1-CDR2 (SEQ ID NO: 14), and VL1-CDR3 (SEQ ID NO: 15);
   b) a second light chain variable domain (VL2) comprising VL2-CDR1 (SEQ ID NO: 18), VL2-CDR2 (SEQ ID NO: 19), and VL2-CDR3 (SEQ ID NO: 20), and
   c) a kappa light chain constant region (Cκ);
wherein the VL1 and the VL2 are connected via a second peptide linker (L2), and each light chain is in the form of VL1-L2-VL2-Cx; and
wherein the first and the second heavy chains form a dimeric Fc region.

Embodiment 2. The DVD-Ig drug conjugate of embodiment 1, wherein the first heavy chain variable domain (VH1) comprises the amino acid sequence of SEQ ID NO: 2; the second heavy chain variable domain (VH2) comprises the amino acid sequence of SEQ ID NO: 7; the first light chain variable domain (VL1) comprises the amino acid sequence of SEQ ID NO: 12; and the second light chain variable domain (VL2) comprises the amino acid sequence of SEQ ID NO: 17.

Embodiment 3. The DVD-Ig drug conjugate of embodiment 1 or 2, wherein the first peptide linker (L1) comprises the amino acid sequence GGGGSGGGGS (SEQ ID NO: 6).

Embodiment 4. The DVD-Ig drug conjugate of any one of embodiments 1-3, wherein the second peptide linker (L2) comprises the amino acid sequence GGSGGGGSG (SEQ ID NO: 16).

Embodiment 5. The DVD-Ig drug conjugate of any one of embodiments 1-4, wherein the IgG1 heavy chain constant region (CH1CH2CH3) comprises the amino acid sequence of SEQ ID NO: 21 or 23.

Embodiment 6. The DVD-Ig drug conjugate of any one of embodiments 1-5, wherein the kappa light chain constant region (Cκ) comprises the amino acid sequence of SEQ ID NO: 22.

Embodiment 7. The DVD-Ig drug conjugate of any one of embodiments 1-6, wherein each of the heavy chains comprise the amino acid sequence of SEQ ID NO: 1 or 24; and wherein each light chain comprises the amino acid sequence of SEQ ID NO: 11.

Embodiment 8. The DVD-Ig drug conjugate of any one of embodiments 1-7, wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering.

Embodiment 9. The DVD-Ig drug conjugate of any one of embodiments 1-8, wherein the Top1i linker-Drugs are conjugated to the cysteines at position C220 of the first and the second heavy chains of the DVD-Ig according to EU numbering.

Embodiment 10. The DVD-Ig drug conjugate of any one of embodiments 1-9, wherein n is 1.

Embodiment 11. The DVD-Ig drug conjugate of any one of embodiments 1-9, wherein n is 2.

Embodiment 12. A dual-variable domain immunoglobulin (DVD-Ig) drug conjugate comprising the following structure: 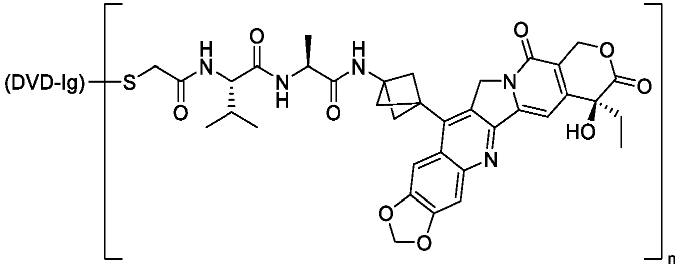
wherein n is 2, and wherein the DVD-Ig binds human STEAP1 and human PSMA, and comprises two identical heavy chains and two identical light chains,
wherein each of the heavy chains comprise the amino acid sequence of SEQ ID NO: 1 or 24; and
wherein each light chain comprises the amino acid sequence of SEQ ID NO: 11;
wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering; and
wherein the Top1i linker-Drugs are conjugated to the cysteines at position C220 of the first and the second heavy chains of the DVD-Ig according to EU numbering.

Embodiment 13. A DVD-Ig that binds human STEAP1 and human PSMA, comprising two identical heavy chains and two identical light chains,
wherein each heavy chain comprises:
   a) a first heavy chain variable domain (VH1) comprising VH1-CDR1 (SEQ ID NO: 3), VH1-CDR2 (SEQ ID NO: 4), and VH1-CDR3 (SEQ ID NO: 5);
   b) a second heavy chain variable domain (VH2) a comprising VH2-CDR1 (SEQ ID NO: 8), VH2-CDR2 (SEQ ID NO: 9), and VH2-CDR3 (SEQ ID NO: 10); and
   c) an IgG1 heavy chain constant region (CH1CH2CH3);
wherein the VH1 and the VH2 are connected via a first peptide linker (L1), and each heavy chain is in the form of VH1-L1-VH2-CH1CH2CH3;
wherein each light chain comprises:
   a) a first light chain variable domain (VL1) comprising VL1-CDR1 (SEQ ID NO: 13), VL1-CDR2 (SEQ ID NO: 14), and VL1-CDR3 (SEQ ID NO: 15);
   b) a second light chain variable domain (VL2) comprising VL2-CDR1 (SEQ ID NO: 18), VL2-CDR2 (SEQ ID NO: 19), and VL2-CDR3 (SEQ ID NO: 20), and
   c) a kappa light chain constant region (Cκ);
wherein the VL1 and the VL2 are connected via a second peptide linker (L2), and each light chain is in the form of VL1-L2-VL2-Cx; and
wherein the first and the second heavy chains form a dimeric Fc region.

Embodiment 14. The DVD-Ig of embodiment 13, wherein the first heavy chain variable domain (VH1) comprises the amino acid sequence of SEQ ID NO: 2; the second heavy chain variable domain (VH2) comprises the amino acid sequence of SEQ ID NO: 7; the first light chain variable domain (VL1) comprises the amino acid sequence of SEQ ID NO: 12; and the second light chain variable domain (VL2) comprises the amino acid sequence of SEQ ID NO: 17.

Embodiment 15. The DVD-Ig of embodiment 13 or 14, wherein the first peptide linker (L1) comprises the amino acid sequence GGGGSGGGGS (SEQ ID NO: 6).

Embodiment 16. The DVD-Ig of any one of embodiments 13-15, wherein the second peptide linker (L2) comprises the amino acid sequence GGSGGGGSG (SEQ ID NO: 16).

Embodiment 17. The DVD-Ig of any one of embodiments 13-16, wherein the IgG1 heavy chain constant region (CH1CH2CH3) comprises the amino acid sequence of SEQ ID NO: 21 or 23.

Embodiment 18. The DVD-Ig of any one of embodiments 13-17, wherein the kappa light chain constant region (Cκ) comprises the amino acid sequence of SEQ ID NO: 22.

Embodiment 19. The DVD-Ig of any one of embodiments 13-18, wherein each of the heavy chains comprise the amino acid sequence of SEQ ID NO: 1 or 24; and wherein each light chain comprises the amino acid sequence of SEQ ID NO: 11.

Embodiment 20. The DVD-Ig of any one of embodiments 13-19, wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering.

Embodiment 21. A DVD-Ig that binds human STEAP1 and human PSMA comprising two identical heavy chains and two identical light chains, wherein each of the heavy chains comprise the amino acid sequence of SEQ ID NO: 1 or 24; and wherein each light chain comprises the amino acid sequence of SEQ ID NO: 11; and wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering.

Embodiment 22. A nucleic acid encoding a heavy chain comprising an amino acid sequence of SEQ ID NO: 1 or 24, and/or a light chain comprising an amino acid sequence of SEQ ID NO: 11.

Embodiment 23. A vector comprising a nucleic acid of embodiment 22.

Embodiment 24. A host cell comprising a nucleic acid of embodiment 22, or a vector of embodiment 23.

All publications, patents, patent applications and other documents cited in this application are hereby incorporated by reference in their entireties for all purposes to the same extent as if each individual publication, patent, patent application or other document were individually indicated to be incorporated by reference for all purposes.

While various specific embodiments have been illustrated and described, it will be appreciated that various changes can be made without departing from the spirit and scope of the invention(s).

## Claims

1. A dual-variable domain immunoglobulin (DVD-Ig) drug conjugate comprising the following structure: 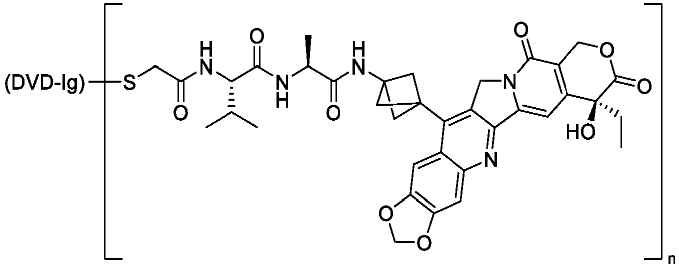
wherein n is 1 or 2,
wherein the DVD-Ig binds human STEAP1 and human PSMA,
wherein the DVD-Ig comprises two identical heavy chains and two identical light chains,
wherein each heavy chain comprises:
a) a first heavy chain variable domain (VH1) comprising VH1-CDR1 (SEQ ID NO: 3), VH1-CDR2 (SEQ ID NO: 4), and VH1-CDR3 (SEQ ID NO: 5);
b) a second heavy chain variable domain (VH2) comprising VH2-CDR1 (SEQ ID NO: 8), VH2-CDR2 (SEQ ID NO: 9), and VH2-CDR3 (SEQ ID NO: 10); and
c) an IgG1 heavy chain constant region (CH1CH2CH3);
wherein the VH1 and the VH2 are connected via a first peptide linker (L1), and each heavy chain is in the form of VH1-L1-VH2-CH1CH2CH3;
wherein each light chain comprises:
a) a first light chain variable domain (VL1) comprising VL1-CDR1 (SEQ ID NO: 13), VL1-CDR2 (SEQ ID NO: 14), and VL1-CDR3 (SEQ ID NO: 15);
b) a second light chain variable domain (VL2) comprising VL2-CDR1 (SEQ ID NO: 18), VL2-CDR2 (SEQ ID NO: 19), and VL2-CDR3 (SEQ ID NO: 20), and
c) a kappa light chain constant region (Cκ);
wherein the VL1 and the VL2 are connected via a second peptide linker (L2), and each light chain is in the form of VL1-L2-VL2-Cx; and
wherein the first and the second heavy chains form a dimeric Fc region.

2. The DVD-Ig drug conjugate of claim 1, wherein
the first heavy chain variable domain (VH1) comprises the amino acid sequence of SEQ ID NO: 2;
the second heavy chain variable domain (VH2) comprises the amino acid sequence of SEQ ID NO: 7;
the first light chain variable domain (VL1) comprises the amino acid sequence of SEQ ID NO: 12; and
the second light chain variable domain (VL2) comprises the amino acid sequence of SEQ ID NO: 17.

3. The DVD-Ig drug conjugate of claim 1 or 2, wherein the first peptide linker (L1) comprises the amino acid sequence GGGGSGGGGS (SEQ ID NO: 6).

4. The DVD-Ig drug conjugate of any one of claims 1-3, wherein the second peptide linker (L2) comprises the amino acid sequence GGSGGGGSG (SEQ ID NO: 16).

5. The DVD-Ig drug conjugate of any one of claims 1-4, wherein the IgG1 heavy chain constant region (CH1CH2CH3) comprises the amino acid sequence of SEQ ID NO: 21 or 23.

6. The DVD-Ig drug conjugate of any one of claims 1-5, wherein the kappa light chain constant region (Cκ) comprises the amino acid sequence of SEQ ID NO: 22.

7. The DVD-Ig drug conjugate of any one of claims 1-6,
wherein each of the heavy chains comprise the amino acid sequence of SEQ ID NO: 1 or 24; and
wherein each light chain comprises the amino acid sequence of SEQ ID NO: 11.

8. The DVD-Ig drug conjugate of any one of claims 1-7, wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering.

9. The DVD-Ig drug conjugate of any one of claims 1-8, wherein the Top1i linker-Drugs are conjugated to the cysteines at position C220 of the first and the second heavy chains of the DVD-Ig according to EU numbering.

10. The DVD-Ig drug conjugate of any one of claims 1-9, wherein n is 1.

11. The DVD-Ig drug conjugate of any one of claims 1-9, wherein n is 2.

12. A dual-variable domain immunoglobulin (DVD-Ig) drug conjugate comprising the following structure: 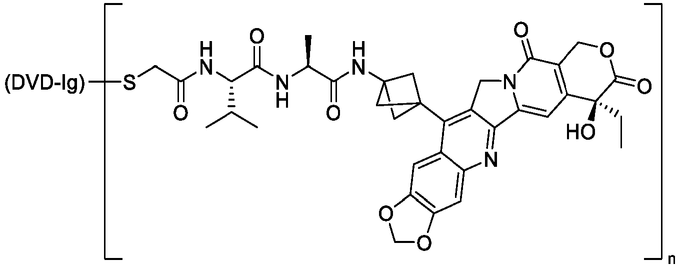
wherein n is 2, and wherein the DVD-Ig binds human STEAP1 and human PSMA, and comprises two identical heavy chains and two identical light chains,
wherein each of the heavy chains comprise the amino acid sequence of SEQ ID NO: 1 or 24; and
wherein each light chain comprises the amino acid sequence of SEQ ID NO: 11;
wherein the first heavy chain and the second heavy chain are connected by two disulfide bridges, and wherein the two disulfide bridges are between the cysteine in position 226 of the first heavy chain and the cysteine in position 226 of the second heavy chain, and between the cysteine in position 229 of the first heavy chain and the cysteine in position 229 of the second heavy chain according to EU numbering; and
wherein the Top1i linker-Drugs are conjugated to the cysteines at position C220 of the first and the second heavy chains of the DVD-Ig according to EU numbering.
